# EUROPEAN PATENT APPLICATION

(11) **EP 4 714 973 A1**
(43) Date of publication of application: **25.03.2026**
(21) Application number: 24806469.3
(22) Date of filing: 10.05.2024
(51) Int. Cl.: C07K 16/28, C07K 16/46

(54) **ANTIBODY SPECIFICALLY BINDING TO CLL1, PREPARATION METHOD THEREFOR, AND USE THEREOF**

(30) Priority: 16.05.2023 CN 202310550914
(71) Applicant: Gracell Bioscience (Shanghai) Co., Ltd., Shanghai 200233 (CN)
(72) Inventor: ZHANG, Hua, Suzhou, Jiangsu 215128 (CN); TANG, Jiaxing, Suzhou, Jiangsu 215128 (CN); SHEN, Lianjun, Suzhou, Jiangsu 215128 (CN); CAO, Wei, Suzhou, Jiangsu 215128 (CN)
(74) Representative: AstraZeneca Intellectual Property
(86) International application number: PCT/CN2024/092299
(87) International publication number: WO 2024/235117

(57) **Abstract**

An antibody specifically binding to CLL1, and a preparation method therefor and a use thereof. Specifically, the present invention provides an anti-CLL1 nanobody or a binding fragment thereof. The antibody has good specificity. Furthermore, the antibody and a CLL1-targeting immune effector cell prepared using the antibody show good therapeutic efficacy in treating or alleviating diseases having CLL1-positive expression.

## Description

### TECHNICAL FIELD

The present invention relates to the field of engineered immunotherapy, and in particular relates to an antibody specifically binding to CLL1, and a preparation method therefor and a use thereof.

### BACKGROUND ART

Acute myeloid leukemia (AML) is a disease exhibiting heterogeneity in both treatment response and survival. It is primarily characterized by differentiation arrest of primitive progenitor cells and immature myeloid cells in bone marrow and peripheral blood, resulting in abnormal proliferation and accumulation of myeloblasts at various differentiation stages, while functionally normal red blood cells, platelets and white blood cells are drastically reduced. AML is generally prevalent across all age groups, and is particularly common in the elderly (over 60 years old), and is the most common type of acute leukemia in adults. AML is primary, refractory, recurrent, and therapeutically lethal, and is a fatal disease. Commonly used treatment methods include chemotherapy and hematopoietic stem cell transplantation.

Chimeric antigen receptor T cell (CAR T) is a novel immunotherapy method targeting a specific antigen on the surface of tumor cells. Currently, it is applied to the development of cell therapies for tumors.

Camelid-derived antibodies (or single-domain antibodies) are advantageous in terms of stable properties, good water solubility, low immunogenicity, stronger affinity, small molecular weight, and stronger tissue penetration than conventional antibodies. Single-domain antibodies can serve as diagnostic tools, and can also be used for the delivery of CAR-T and targeted drugs.

Therefore, there is an urgent need in the art to develop a novel CAR-T with stronger affinity, higher specificity, and/or lower immunogenicity for the treatment of acute myeloid leukemia.

### SUMMARY OF THE INVENTION

The objective of the present invention is to provide an antibody that has high affinity and high biological activity and can specifically recognize a CLL1 antigen, and a use thereof.

In a first aspect of the present invention, a nanobody that specifically binds to CLL1 is provided. Complementary determining regions (CDRs) of a VHH chain of the nanobody comprise the following CDR1, CDR2, and CDR3:
(a) CDR1: the sequence thereof is as shown in SEQ ID NO: 14, 11, or 20;
(b) CDR2: the sequence thereof is as shown in SEQ ID NO: 12, 15, 17, or 19; and
(c) CDR3: the sequence thereof is as shown in SEQ ID NO: 13, 16, 18, 21, or 22.

In another preferred embodiment, the complementary determining regions (CDRs) of the VHH chain of the nanobody are selected from a group consisting of:
(Y1) CDR1 as shown in SEQ ID NO: 14, CDR2 as shown in SEQ ID NO: 19, and CDR3 as shown in SEQ ID NO: 16;
(Y2) CDR1 as shown in SEQ ID NO: 14, CDR2 as shown in SEQ ID NO: 12, and CDR3 as shown in SEQ ID NO: 22;
(Y3) CDR1 as shown in SEQ ID NO: 14, CDR2 as shown in SEQ ID NO: 15, and CDR3 as shown in SEQ ID NO: 16;
(Y4) CDR1 as shown in SEQ ID NO: 14, CDR2 as shown in SEQ ID NO: 17, and CDR3 as shown in SEQ ID NO: 18;
(Y5) CDR1 as shown in SEQ ID NO: 14, CDR2 as shown in SEQ ID NO: 19, and CDR3 as shown in SEQ ID NO: 16;
(Y6) CDR1 as shown in SEQ ID NO: 11, CDR2 as shown in SEQ ID NO: 12, and CDR3 as shown in SEQ ID NO: 13; and
(Y7) CDR1 as shown in SEQ ID NO: 20, CDR2 as shown in SEQ ID NO: 12, and CDR3 as shown in SEQ ID NO: 21.

In another preferred embodiment, CDR1, CDR2, and CDR3 are separated by framework regions FR1, FR2, FR3, and FR4 of the VHH chain.

In another preferred embodiment, the nanobody that specifically binds to CLL1 includes a humanized antibody, a camelid-derived antibody, and a chimeric antibody.

In another preferred embodiment, the amino acid sequence of the nanobody that specifically binds to CLL1 is as shown in any one of SEQ ID NOs: 1-10.

In another preferred embodiment, the amino acid sequence of the VHH chain of the nanobody is selected from a group consisting of: SEQ ID NOs: 1-10, or a combination thereof.

In another preferred embodiment, the CDRs of the VHH chain of the nanobody comprise an amino acid sequence having at least 80%, preferably at least 90%, more preferably at least 95%, and even more preferably at least 99% sequence identity to any one of SEQ ID NOs: 1-10.

In another preferred embodiment, the amino acid sequence of the CDRs of the VHH chain of the nanobody comprises one or more amino acid substitutions, preferably conservative amino acid substitutions, compared with any one of SEQ ID NOs: 1-10.

In another preferred embodiment, any one of the aforementioned amino acid sequences further includes a derivative sequence optionally having at least one (e.g., 1 to 3, preferably 1 to 2, and more preferably 1) added, deleted, modified, and/or substituted amino acid and retaining the ability to specifically bind CLL1.

In another preferred embodiment, the CLL1 is CLL1 from a human or a non-human mammal.

In another preferred embodiment, the CLL1 is CLL1 from a human, a mouse, a rat, or a non-human primate (such as a monkey).

In a second aspect of the present invention, a multivalent antibody or multi-epitope-targeting antibody that specifically binds to CLL1 is provided, the multivalent antibody or multi-epitope-targeting antibody comprising at least one antibody component that targets a CLL1 epitope, the antibody component being the anti-CLL1 nanobody according to the first aspect of the present invention.

In another preferred embodiment, the anti-CLL1 multivalent antibody or multi-epitope-targeting antibody comprises one or more anti-CLL1 nanobodies.

In another preferred embodiment, the anti-CLL1 antibody comprises a monomer, a dimer (bivalent antibody), a tetramer (tetravalent antibody), and/or a multimer (multivalent antibody).

In another preferred embodiment, the anti-CLL1 antibody comprises one or more VHH chains having the amino acid sequence as shown in any one of SEQ ID NOs: 1-10.

In another preferred embodiment, the anti-CLL1 antibody comprises two VHH chains having the amino acid sequence as shown in any one of SEQ ID NOs: 1-10.

In another preferred embodiment, the antibody is selected from: an animal-derived antibody, a chimeric antibody, a humanized antibody, or a combination thereof.

In a third aspect of the present invention, a recombinant protein is provided, the recombinant protein comprising:
(i) the nanobody that specifically binds to CLL1 according to the first aspect of the present invention, or the multivalent antibody or multi-epitope-targeting antibody that specifically binds to CLL1 according to the second aspect of the present invention; and
(ii) an optional tag sequence that facilitates expression and/or purification.

In another preferred embodiment, the antibody is a multivalent antibody.

In another preferred embodiment, the tag sequence includes an Fc tag, an HA tag, a Flag tag, and a 6His tag.

In another preferred embodiment, the Fc tag comprises mlgG2aFc.

In another preferred embodiment, the recombinant protein specifically binds to a CLL1 protein.

In a fourth aspect of the present invention, a chimeric antigen receptor (CAR) fusion protein is provided, wherein the chimeric antigen receptor (CAR) fusion protein comprises, from the N-terminus to the C-terminus:
(i) an antigen-binding domain that specifically binds to CLL1, the antigen-binding domain comprising the nanobody according to the first aspect of the present invention;
(ii) a transmembrane domain;
(iii) at least one co-stimulatory domain; and
(iv) an activation domain.

In another preferred embodiment, the antigen-binding domain is monovalent or multivalent.

In another preferred embodiment, the antigen-binding domain is from the nanobody according to the first aspect of the present invention or the recombinant protein according to the third aspect of the present invention.

In another preferred embodiment, the CAR has the structure shown in Formula Ia:

L-VHH-F-H-TM-C-CD3ζ (la)

where,
each "-" is independently a linker peptide or peptide bond;
L is a signal peptide sequence;
VHH is the antigen-binding domain that specifically binds to CLL1;
F is none or a Flag tag;
H is a hinge region;
TM is a transmembrane domain;
C is a co-stimulatory signal domain; and
CD3ζ is a cytoplasmic signal transduction sequence (including wild-type, or mutant/modification thereof) derived from CD3ζ.

In another preferred embodiment, L is a signal peptide of a protein selected from a group consisting of: CD28, 4-1BB, GM-CSF, CD3, CD8a, or a combination thereof.

In another preferred embodiment, the L is a signal peptide derived from CD8.

In another preferred embodiment, the L comprises an amino acid sequence as shown in SEQ ID NO: 27.

In another preferred embodiment, the amino acid sequence of VHH is as shown in any one of SEQ ID NOs: 1-10.

In another preferred embodiment, H comprises a hinge region of CD28.

In another preferred embodiment, TM includes a transmembrane region derived from CD28.

In another preferred embodiment, the amino acid sequence of H and TM comprises an amino acid sequence as shown in SEQ ID NO: 29.

In another preferred embodiment, C is a transmembrane region of a protein selected from a group consisting of: CD28, 4-1BB, CD8a, or a combination thereof.

In another preferred embodiment, C includes a co-stimulatory signal molecule derived from 4-1BB.

In another preferred embodiment, C comprises an amino acid sequence as shown in SEQ ID NO: 30.

In another preferred embodiment, CD3ζ comprises an amino acid sequence as shown in SEQ ID NO: 31.

In another preferred embodiment, the CAR fusion protein has an amino acid sequence as shown in any one of SEQ ID NOs: 32-41.

In a fifth aspect of the present invention, an antibody-drug conjugate is provided, the antibody-drug conjugate comprising:
(a) the nanobody according to the first aspect of the present invention, the multivalent antibody or multi-epitope-targeting antibody according to the second aspect of the present invention, or the recombinant protein according to the third aspect of the present invention; and
(b) a conjugated moiety coupled to the antibody moiety, the conjugated moiety being selected from a group consisting of: a detectable label, a drug, a toxin, a cytokine, a radionuclide, an enzyme, or a combination thereof.

In another preferred embodiment, the antibody moiety is coupled to the conjugated moiety via a chemical bond or a linker.

In a sixth aspect of the present invention, a polynucleotide is provided, the polynucleotide encoding a protein selected from a group consisting of: the nanobody according to the first aspect of the present invention, the multivalent antibody or multi-epitope-targeting antibody according to the second aspect of the present invention, the recombinant protein according to the third aspect of the present invention, or the CAR fusion protein according to the fourth aspect of the present invention.

In a seventh aspect of the present invention, an expression vector is provided, the expression vector comprising the polynucleotide according to the sixth aspect of the present invention.

In another preferred embodiment, the expression vector is selected from a group consisting of: DNA, RNA, viral vectors, plasmids, transposons, other gene transfer systems, or a combination thereof.

In another preferred embodiment, the expression vector is a lentiviral vector.

In an eighth aspect of the present invention, a host cell is provided, the host cell comprising the expression vector according to the seventh aspect of the present invention, or having the polynucleotide according to the sixth aspect of the present invention integrated in a genome thereof, or expressing the nanobody according to the first aspect of the present invention, the multivalent antibody or multi-epitope-targeting antibody according to the second aspect of the present invention, the recombinant protein according to the third aspect of the present invention, or the CAR fusion protein according to the fourth aspect of the present invention.

In another preferred embodiment, the cell is an isolated cell, and/or the cell is a genetically engineered cell.

In another preferred embodiment, the cell is a mammalian cell.

In another preferred embodiment, the cell is a T cell.

In another preferred embodiment, the host cell is an engineered immune cell.

In another preferred embodiment, the engineered immune cell is selected from a group consisting of:
(i) chimeric antigen receptor αβT cells (CAR-T cells);
(ii) chimeric antigen receptor γδT cells (CAR-T cells);
(iii) chimeric antigen receptor NKT cells (CAR-NKT cells);
(iv) chimeric antigen receptor NK cells (CAR-NK cells); and
(v) chimeric antigen receptor macrophages.

In a ninth aspect of the present invention, a method for preparing an engineered immune cell is provided, comprising the following step: transducing, into a T cell or an NK cell, the nucleic acid molecule according to the sixth aspect of the present invention or the vector according to the seventh aspect of the present invention, so as to obtain an engineered immune cell, the engineered immune cell expressing the CAR fusion protein according to the fourth aspect of the present invention.

In another preferred embodiment, the method further comprises the step of: testing the function and efficacy of the obtained engineered immune cell.

In a tenth aspect of the present invention, a formulation is provided, the formulation comprising the nanobody according to the first aspect of the present invention, the multivalent antibody or multi-epitope-targeting antibody according to the second aspect of the present invention, the recombinant protein according to the third aspect of the present invention, the CAR fusion protein according to the fourth aspect of the present invention, the vector according to the seventh aspect of the present invention, or the host cell according to the eighth aspect of the present invention, and a pharmaceutically acceptable carrier, diluent, or excipient.

In another preferred embodiment, the host cell is an engineered immune cell.

In an eleventh aspect of the present invention, a pharmaceutical composition is provided, the pharmaceutical composition comprising:
(i) the nanobody according to the first aspect of the present invention, the multivalent antibody or multi-epitope-targeting antibody according to the second aspect of the present invention, the recombinant protein according to the third aspect of the present invention, or an immune cell that expresses the CAR fusion protein according to the fourth aspect of the present invention; and
(ii) a pharmaceutically acceptable carrier.

In another preferred embodiment, the conjugated moiety of the immunoconjugate is a drug, a toxin, and/or a therapeutic isotope.

In another preferred embodiment, the pharmaceutical composition further comprises other drugs for treating immune system diseases or tumor diseases.

In another preferred embodiment, the pharmaceutical composition is used to prepare a drug for the prevention and/or treatment of a CLL1-related disease or condition.

In another preferred embodiment, the CLL1-related disease or condition is selected from a group consisting of: acute myeloid leukemia (AML), chronic myeloid leukemia (CML), myelodysplastic syndrome (MDS), or a combination thereof.

In a twelfth aspect of the present invention, a kit is provided, the kit comprising the nucleic acid molecule according to the sixth aspect of the present invention, the recombinant protein according to the third aspect of the present invention, or the vector according to the seventh aspect of the present invention.

In another preferred embodiment, the kit is used to prepare an immune cell that expresses the receptor CAR fusion protein according to the fourth aspect of the present invention.

In a thirteenth aspect of the present invention, provided is a use of the nanobody according to the first aspect of the present invention, the multivalent antibody or multi-epitope-targeting antibody according to the second aspect of the present invention, the recombinant protein according to the third aspect of the present invention, or an immune cell that expresses the CAR fusion protein according to the fourth aspect of the present invention, the use being for the preparation of a drug for preventing and/or treating a CLL1-related cancer or tumor.

In a fourteenth aspect of the present invention, a method for treating a disease is provided, comprising administering to a subject in need of treatment: the nanobody according to the first aspect of the present invention, the multivalent antibody or multi-epitope-targeting antibody according to the second aspect of the present invention, the recombinant protein according to the third aspect of the present invention, or an immune cell that expresses the CAR fusion protein according to the fourth aspect of the present invention.

In another preferred embodiment, the disease is a tumor having CLL1-positive expression.

It should be understood that within the scope of the present invention, the above-described technical features of the present invention and the technical features specifically described below (as in Examples) can be combined with each other to form new or preferred technical solutions. Due to space limitation, such technical solutions will not be elaborated herein.

It should be understood that within the scope of the present invention, the above-described technical features of the present invention and the technical features specifically described below (as in Examples) can be combined with each other to form new or preferred technical solutions. Due to space limitation, such technical solutions will not be elaborated herein.

### BRIEF DESCRIPTION OF THE DRAWINGS

Fig. 1 shows the expression of CLL1 on the surface of target cells.
Fig. 2 shows the CAR positive rate on the surface of CLL1 C-CAR-T cells.
Fig. 3 shows the cytotoxicity of CLL1 C-CAR-T against wild-type Hela cells and CLL1-overexpressing Hela cells (RTCA assay).
Fig. 4 shows the cytotoxicity of CLL1 C-CAR-T against K562, KG1, and HL60 cells (luciferase assay).
FIG. 5 shows the recognition of antigens on the surface of target cells by the CLL1 antibody.
Fig. 6 shows the CAR positive rate on the surface of CLL1 F-CAR T cells.
Fig. 7 shows a schematic flow chart of flow cytometry analysis of in vitro multi-round killing by CAR-T cells.
Fig. 8 shows the multi-round killing results of CLL1 C-CAR T and CLL1 F-CAR T cells against HL60 cells.
Fig. 9 shows the multi-round killing results of CLL1 C-CAR T and CLL1 F-CAR T cells against KG1 cells.
Fig. 10 shows the CAR positive rate after CAR-NK92 sorting.
Fig. 11 shows the cytotoxicity of CAR-NK92 against tumor cells (luciferase assay).
Fig. 12 shows the multi-round killing results of CAR-N92K.

### DETAILED DESCRIPTION OF PREFERRED EMBODIMENTS

After extensive and in-depth research, the present inventors unexpectedly obtained for the first time an anti-CLL1 antibody having excellent affinity and high anti-tumor activity. Specifically, via extensive screening, the present inventors obtained for the first time multiple nanobodies specifically binding to CLL1. On the basis of the nanobodies, recombinant antibodies and humanized antibodies, as well as CLL1-targeting chimeric antigen receptors, were further prepared in the present invention. Based on an alpaca heavy chain antibody that specifically binds to CLL1, the present invention provides CAR-Ts having higher affinity, stronger specificity for target antigens, and lower immunogenicity compared to conventional antibodies. The CAR-T cells of the present invention have excellent target cell-killing activity and in vivo tumorsuppressing effects. On that basis, the present invention has been completed.

Specifically, in the present invention, a human-derived CLL1 antigen protein was used to immunize alpacas to obtain a high-quality immune nanobody gene library. Then, the CLL1 protein molecule were coupled to an ELISA plate to display the correct spatial structure of the CLL1 protein. Then, using the antigen in this form, the immune nanobody gene library (alpaca heavy chain antibody phage display gene library) was screened by phage display technology, thereby obtaining nanobody genes specific for the CLL1 protein. The genes were then transferred into E. coli to obtain a nanobody strain that can be efficiently expressed and has high specificity in E. coli.

### Terms

In order to more easily understand the present disclosure, specific terms are first defined. As used in the present application, unless otherwise expressly indicated herein, each of the following terms shall have the meaning given below. Additional definitions are set forth throughout the application.

The term "about" may refer to a value or composition that is within an acceptable error range for a particular value or composition as determined by one of ordinary skill in the art, which will depend in part on how the value or composition is measured or determined. For example, as used herein, the expression "about 100" includes 99 and 101 and all values therebetween (such as 99.1, 99.2, 99.3, 99.4, etc.).

As used herein, the term "contain" or "include (comprise)" may be open-ended, semi closed-ended, and close-ended. In other words, the term also includes "substantially composed of" or "composed of'.

Sequence identity is determined by comparing two aligned sequences along a predetermined comparison window (the window can be 50%, 60%, 70%, 80%, 90%, 95%, or 100% of the length of a reference nucleotide sequence or protein), and determining the number of positions where the same residue appears. Typically, sequence identity is expressed as a percentage. Methods for measuring sequence identity of nucleotide sequences is well-known for those skilled in the art.

As used herein, the terms "heavy chain variable region" and "VH" can be used interchangeably.

As used herein, the terms "variable region" and "complementary determining region (CDR)" can be used interchangeably.

In the present invention, the term "antibody of the present invention", "single-domain antibody of the present invention", "protein of the present invention" or "polypeptide of the present invention" can be used interchangeably, and all refer to an antibody that specifically binds to CLL1, such as a protein or polypeptide having a heavy chain variable region (such as amino acid sequences set forth in SEQ ID NOs: 1-10). They may or may not contain a starting methionine.

### CLL1

C-type lectin-like-1 (CLL1), also known as C-type lectin domain family 12 member A (CLEC12A) or CD371, is a type II transmembrane protein. CLL1 is a glycoprotein receptor and a member of the large family of C-type lectin-like receptors involved in immunomodulation. CLL1 is expressed on hematopoietic cells, mainly on innate immune cells including monocytes, DCs and granulocytes, and on bone marrow progenitor cells.

CLL-1 is also found in acute myeloid leukemia (AML) blasts and leukemic stem cells (such as CD34⁺/CD38⁻). CLL1 may also be expressed on the surface of cells of other myeloid leukemias, such as acute myeloid leukemia (AML), chronic myeloid leukemia (CML), and myelodysplastic syndrome (MDS).

Studies have found that CLL1 is expressed on AML stem cells (CD34⁺/CD38⁻) and a small fraction of hematopoietic progenitor cells (CD34⁺/CD38⁺ or CD34⁺/CD33⁺), but is not expressed on normal hematopoietic stem cells (CD34⁺/CD38⁻ or CD34⁺/CD33⁻).

Therefore, single domain antibodies and CAR engineered immune cells (such as CAR-T cells) targeting CLL1 can be used to treat myeloid leukemias with high CLL1 expression, representative examples including but not limited to: acute myeloid leukemia (AML), chronic myeloid leukemia (CML), and myelodysplastic syndrome (MDS).

### Antibody

As used herein, the term "antibody" refers to an immunoglobulin, which is a tetrapeptide-chain structure formed of two identical heavy chains and two identical light chains connected by inter-chain disulfide bonds.

As used herein, the terms "single-domain antibody (VHH)" and "nanobody" have the same meaning, and refer to a variable region of a heavy chain of a cloned antibody. A single-domain antibody (VHH) consisting of only one heavy chain variable region is constructed, and is the smallest antigen-binding fragment having full functions. Generally, after an antibody naturally lacking in the light chain and the heavy chain constant region 1 (CH1) is first obtained, the variable region of the heavy chain of the antibody is cloned to construct a single-domain antibody (VHH) consisting of only one heavy chain variable region.

Existing antibody numbering schemes include:
1. The Kabat scheme (Kabat et al., 1991) is based on the location of regions of high sequence variation between sequences of the same domain type. Antibody heavy (VH) and light (Vλ and Vκ) variable domains are numbered differently.
2. Chothia's scheme (Al-Lazikani, 1997) is the same as Kabat's but corrects where an insertion is annotated around the first VH complementarity determining region (CDR) so that it corresponds to a structural loop. Similarly, the Enhanced Chothia scheme (Abhinandan and Martin, 2008) makes further structural corrections of indel positions.
3. In contrast to these Kabat-like schemes, IMGT (Lefranc, 2003) and AHo (Honegger and Plückthun, 2001) both define unique schemes for antibody and T cell receptor (TCR) (Vα and Vβ) variable domains. Thus, equivalent residue positions can easily be compared between domain types. IMGT and AHo differ in the number of positions they annotate (128 and 149 respectively) and where they consider indels to occur.

Immunoglobulins differ in the composition and arrangement order of the amino acids of the heavy chain constant regions, so their antigenicity is also different. Accordingly, immunoglobulins may be divided into five classes, or immunoglobulin isotypes, namely IgM, IgD, IgG, IgA, and IgE, their corresponding heavy chains being a µ chain, a δ chain, a γ chain, an α chain, and an ε chain, respectively. Each class of Ig may be subdivided into different subclasses according to the differences in the amino acid composition of the hinge region and the number and position of the heavy chain disulfide bond. For example, IgG may be divided into IgG1, IgG2, IgG3, and IgG4. Light chains may be a κ chain or a λ chain depending on the constant region thereof. Each of the five classes of Ig may have a κ chain or a λ chain. The subunit structures and three-dimensional configurations of different classes of immunoglobulin are well-known to those skilled in the art.

The light chains of the antibody according to the present invention may further comprise a light chain constant region, the light chain constant region comprising a human or murine κ chain, λ chain, or variant thereof.

In the present invention, the heavy chains of the antibody according to the present invention may further comprise heavy chain constant regions, the heavy chain constant regions comprising human or murine IgG1, IgG2, IgG3, IgG4 or variants thereof. The sequences of about 110 amino acids towards the N terminus of the heavy chains and the light chains of the antibody vary greatly, and constitute variable regions (Fv regions); the sequences of the other amino acids towards the C terminus are relatively stable, and constitute constant regions. The variable regions include three hypervariable regions (HVRs) and four framework regions (FRs) having a relatively conserved sequence. The three hypervariable regions determine the specificity of the antibody, and are also called complementarity determining regions (CDRs). Each light chain variable region (LCVR) and each heavy chain variable region (HCVR) consist of three CDRs and four FRs, which are arranged in the order of FR1, CDR1, FR2, CDR2, FR3, CDR3, and FR4 from the amino terminus to the carboxyl terminus. The three CDRs of the light chains are designated as LCDR1, LCDR2, and LCDR3; the three CDRs of the heavy chains are designated as HCDR1, HCDR2, and HCDR3.

The term "murine antibody" in the present invention refers to an anti-CLL1 monoclonal antibody prepared according to knowledge and skills in the art. During preparation, a test subject is injected with the CLL1 antigen, and then a hybridoma expressing the antibody having the desired sequence or functional characteristics is isolated. In a preferred embodiment of the present invention, the murine CLL1 antibody or an antigen-binding fragment thereof may further comprise a light chain constant region of a murine κ or λ chain or a variant thereof, or may further comprise a heavy chain constant region of murine IgG1, IgG2, or IgG3, or a variant thereof.

The term "chimeric antibody" refers to an antibody formed by fusing variable regions of a murine antibody with constant regions of a human antibody. The chimeric antibody can alleviate an immune response induced by the murine antibody.

The term "humanized antibody", also known as CDR-grafted antibody, refers to an antibody produced by grafting murine CDR sequences into human antibody variable region frameworks, that is, different types of human germline antibody framework sequences. The humanized antibody can overcome a heterogeneous reaction induced by a chimeric antibody which carries a large amount of murine protein components. Such framework sequences can be obtained from public DNA databases or published references that include germline antibody gene sequences. In order to avoid the decrease in activity resulting from the decrease

of immunogenicity, the human antibody variable region framework sequences can be subjected to minimal reverse mutations or back mutations to maintain the activity.

The term "antigen-binding fragment of an antibody" (or abbreviated "antibody fragment") refers to one or more fragments of an antibody that retain the ability to specifically bind to an antigen (e.g., CLL1). It has been shown that a fragment of a full-length antibody can be used to perform the antigen-binding function of the antibody. Examples of binding fragments included in the term "antigen-binding fragment of an antibody" include:
(i) Fab fragment, a monovalent fragment consisting of VL, VH, CL, and CH1 domains;
(ii) F(ab')₂ fragment, a divalent fragment comprising two Fab fragments connected by a disulfide bridge on the hinge region;
(iii) Fd fragment, consisting of VH and CH1 domains; and
(iv) Fv fragment, consisting of the VH and VL domains of a single arm of an antibody.

An Fv antibody comprises a heavy chain variable region and a light chain variable region, but does not comprise a constant region. The Fv antibody is the smallest antibody fragment having all antigen-binding sites. Generally, an Fv antibody further comprises a polypeptide linker between the VH and VL domain, and can form the structure required for antigen binding.

The term "CDR" refers to one of the six hypervariable regions, within the variable domains of an antibody, that mainly contribute to antigen binding. One of the most commonly used definitions of the six CDRs is provided by Kabat E.A et al., (1991) Sequences of proteins of immunological interest. NIH Publication, No. 91-3242

The term "epitope" or "antigenic determinant" refers to a site on an antigen to which an immunoglobulin or antibody specifically binds (e.g., a specific site on the CLL1 molecule). An epitope typically comprises at least 3, 4, 5, 6, 7, 8, 9, 10, 11, 12, 13, 14, or 15 consecutive or non-consecutive amino acids in a unique spatial conformation.

The terms "specific binding", "selective binding", "selectively bind", and "specifically bind" refer to binding of an antibody to a predetermined epitope on an antigen. Typically, an antibody binds with an affinity (KD) of approximately

less than 10⁻⁷ M, such as approximately less than 10⁻⁸ M, 10⁻⁹ M or 10¹⁰ M or less.

The term "competitive binding" refers to an antibody that recognizes the same epitope (also referred to as antigenic determinant) or a portion of the same epitope on an extracellular region of CLL1 and binds to the antigen as the monoclonal antibody of the present invention does. An antibody that binds to the same epitope as the monoclonal antibody of the present invention does refers to an antibody that recognizes and binds to an amino acid sequence of CLL1 that is recognized by the monoclonal antibody of the present invention.

The term "KD" or "Kd" refers to the dissociation equilibrium constant of a particular antibody-antigen interaction. Typically, the antibody of the present invention binds to CLL1 with a dissociation equilibrium constant (KD) of less than about 10⁻⁷ M, such as less than about 10⁻⁸ M, 10⁻⁹ M, or 10⁻¹⁰ M, or even less.

As used herein, the term "antigenic determinant" refers to a three-dimensional spatial site on an antigen that is discontinuous and recognized by the antibody or antigen-binding fragment of the present invention.

The present invention comprises not only the entire antibody, but also a fragment of the antibody or a fusion protein formed by the antibody with another sequence, the fragment or fusion protein having immune activity. Therefore, the present invention also includes fragments, derivatives, and analogues of the antibody.

In the present invention, the antibody includes murine, chimeric, humanized, or fully human antibodies prepared using techniques familiar to those skilled in the art. A recombinant antibody, such as a chimeric and humanized monoclonal antibody, comprises both human and non-human portions, and can be prepared using DNA recombination techniques well-known in the art.

As used herein, the term "monoclonal antibody" refers to an antibody secreted by a clone derived from a single cell. The monoclonal antibody is highly specific and targets a single antigenic epitope. The cell may be a eukaryotic, prokaryotic or phage clonal cell line.

In the present invention, the antibody may be monospecific, bispecific, trispecific, or of greater multispecificity.

In the present invention, the antibody of the present invention further includes conserved variants thereof. The conserved variants refer to, compared with the amino acid sequence of the antibody of the present invention, polypeptides formed by replacing at most 10, preferably at most 8, more preferably at most 5, and most preferably 3 amino acids with amino acids having similar or close properties. These conservative variant polypeptides are preferably produced by performing amino acid substitutions according to Table A below.

**Table A**

| Initial residue | Representative substitution(s) | Preferred substitution |
|---|---|---|
| Ala (A) | Val; Leu; Ile | Val |
| Arq (R) | Lys; Gln; Asn | Lys |
| Asn (N) | Gln; His; Lys; Arq | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Humanized anti-CLL1 antibody

The present invention provides a humanized anti-CLL1 antibody. Specifically, the present invention provides a humanized antibody having high specificity and high affinity to CLL1. The humanized antibody comprises a heavy chain and a light chain, the heavy chain comprising an amino acid sequence of a heavy chain variable region (VH), and the light chain comprising an amino acid sequence of a light chain variable region (VL).

Generally, there are two types of human framework region that could be selected for antibody humanization: one is from a known mature antibody, and the other is from a human Germline sequence. Framework regions of a known mature antibody typically comprise somatic cell mutation sites, possibly bringing about potential immunogenicity. Compared to a mature antibody, framework regions of a human Germline sequence theoretically have lower immunogenicity; moreover, the framework regions have greater structural flexibility and plasticity, and are easy to accept different CDRs. There is a bias with respect to the usage frequency of human antibody Germline genes in the human body. An antibody humanized by selecting and using a Germline framework region having a high usage frequency has advantages such as low immunogenicity, high expression level, and stable structure.

In a preferred embodiment of the present invention, during humanization, multiple factors (including similarity, usage frequency in the human body, etc.) are considered at the same time, and after extensive experimental screening, framework regions having preferred sequences are selected for humanization. Human antibody Germline framework regions are selected and used to perform CDR transplantation, and the humanized antibodies thus constructed have a more stable structure, a high expression level, a low immunogenicity, and a higher druggability.

In another preferred embodiment, the heavy chain constant region and/or light chain constant region may be a humanized heavy chain constant region or light chain constant region. More preferably, the humanized heavy chain constant region or light chain constant region is the heavy chain constant region of human IgG1, IgG2, etc., or the human kappa or lambda light chain constant region.

In another preferred embodiment, the sequence formed by adding, deleting, modifying, and/or substituting at least one amino acid sequence is preferably an amino acid sequence having least 80%, preferably at least 85%, more preferably at least 90%, and most preferably at least 95% homology.

The antibody of the present invention may be a double-stranded or single-stranded antibody, and may preferably be a fully humanized antibody.

The antibody derivatives according to the present invention may be single-chain antibodies, and/or antibody fragments, such as Fab, Fab', (Fab')2, or other known antibody derivatives in the art, as well as any one or more of IgA, IgD, IgE, IgG, and IgM antibodies or other subtypes thereof.

The antibody of the present invention may be a humanized antibody or a CDR-grafted and/or modified antibody targeting CLL1.

In the above content of the present invention, the number of the amino acids added, deleted, modified and/or substituted is preferably no more than 40%, more preferably no more than 35%, more preferably 1% to 33%, more preferably 5% to 30%, more preferably 10%to 25%, and more preferably 15% to 20% of the total number of amino acids in the initial amino acid sequence.

### Preparation of antibody

Any method suitable for producing monoclonal antibodies may be used to produce the CLL1 antibody of the present invention. For example, an animal may be immunized with a conjugated or naturally occurring CLL1 protein or a fragment thereof. Suitable immunization methods may be used, including adjuvants, immunostimulants, and repeated booster immunization, and one or more means can be used.

Any suitable form of CLL1 may serve as an immunogen (antigen) for generating a non-human antibody specific to CLL1, and the antibody is screened for biological activities. The immunogen may be used alone or in combination with one or more immunogenic enhancers known in the art. The immunogen may be purified from a natural source, or produced in a genetically modified cell. The DNA encoding the immunogen may either be genomic or non-genomic (such as cDNA) in terms of source. A suitable genetic vector may be used to express the DNA encoding the immunogen. The vector includes but is not limited to adenovirus vectors, baculovirus vectors, plasmids, and nonviral vectors.

The humanized antibody may be selected from any type of immunoglobulin, including IgM, IgD, IgG, IgA, and IgE. Likewise, any type of light chain may be used in the compound and method herein. Specifically, the κ or λ chain or variant thereof may be used in the compound and method of the present invention.

The sequence of the DNA molecule for the antibody or fragment thereof of the present invention may be obtained using conventional techniques, such as PCR amplification or genomic library screening, among other methods. In addition, the coding sequences of the light and heavy chains may be fused together to form a single-chain antibody.

Once relevant sequences are obtained, a recombination method may be used to obtain the relevant sequences in large quantities. This typically involves cloning the relevant sequences into a vector, then transferring the vector into a cell, and then isolating the relevant sequences from the proliferated host cell by a conventional method so as to obtain the relevant sequences.

In addition, the relevant sequences may also be synthesized artificially, especially in the case that the fragments are short in length. In general, a fragment having a very long sequence may be obtained by first synthesizing a plurality of small fragments and then joining same. The DNA sequence may then be introduced into various existing DNA molecules (or such as vectors) and cells known in the art.

The term "nucleic acid molecule" refers to DNA molecules and RNA molecules. The nucleic acid molecule may be single-stranded or double-stranded, but preferably double-stranded DNA. When a nucleic acid is placed in a functional relationship with another nucleic acid sequence, the nucleic acid is "operably linked." For example, if a promoter or enhancer affects the transcription of a coding sequence, the promoter or enhancer is operably linked to the coding sequence.

The term "vector" refers to a nucleic acid molecule capable of transporting another nucleic acid to which it has been linked. In one embodiment, the vector is a "plasmid", which refers to a circular double-stranded DNA loop into which an additional DNA segment may be linked.

The present invention further relates to vectors comprising the above-mentioned suitable DNA sequence and a suitable promoter or control sequence. These vectors can be used to transform an appropriate host cell to enable the host cell to express the protein.

The term "host cell" refers to a cell into which an expression vector has been introduced. The host cell may be a prokaryotic cell, such as a bacterial cell; or a lower eukaryotic cell, such as a yeast cell; or a higher eukaryotic cell, such as a plant or animal cell (e.g., a mammalian cell).

The step of transforming the host cell with the recombinant DNA according to the present invention may be performed using techniques well known in the art. The obtained transformant may be cultured using a conventional method, whereby the transformant expresses the polypeptide encoded by the gene of the present invention. Culture is carried out in a conventional medium under suitable conditions according to the host cell used.

Generally, the host cell obtained from the transformation is cultured under conditions suitable for the expression of the antibody of the present invention. Then, a conventional immunoglobulin purification step is performed by conventional isolation and purification means well known to a person skilled in the art, such as protein A-Sepharose, hydroxyapatite chromatography, gel electrophoresis, dialysis, ion exchange chromatography, hydrophobic chromatography, molecular sieve chromatography or affinity chromatography.

The monoclonal antibody obtained may be identified by conventional means. For example, the binding specificity of the monoclonal antibody may be determined using immunoprecipitation or an in vitro binding assay (e.g., radioimmunoassay (RIA) or enzyme-linked immunosorbent assay (ELISA)).

### Antibody formulations

An antibody has different stabilities in different formulation buffer solutions, manifested by a change in charge heterogeneity, degradation of the antibody molecule, polymerization, etc. These changes in quality properties are related to the physical and chemical properties of the antibody per se. Therefore, in the development of antibody drugs, it is necessary to screen for formulation buffer solutions that are suitable for different antibodies according to the physical and chemical properties of the antibodies. Currently, commonly used antibody formulation buffer systems include phosphate buffers, citric acid buffers, histidine buffers, etc. At the same time, different concentrations of salt ions or excipients such as sorbitol, trehalose, and sucrose as well as suitable amounts of surfactants such as Tween may be added according to the properties of the antibody so as to maintain the stability of the antibody.

### Pharmaceutical composition

The present invention further provides a composition. In a preferred embodiment, the composition is a pharmaceutical composition containing the aforementioned antibody or an active fragment, fusion protein or ADC thereof, or a corresponding CAR-T cell, and a pharmaceutically acceptable carrier. Generally, these substances may be formulated in a non-toxic, inert, and pharmaceutically acceptable aqueous carrier medium, in which the pH is typically about 5 to 8, and preferably about 6 to 8, although the pH value may vary depending on the properties of the substances formulated and the disease to be treated. The formulated pharmaceutical composition may be administered through conventional means, including (but not limited to) intratumoral, intraperitoneal, intravenous, or topical administration.

The antibody of the present invention may also be intracellularly expressed by a nucleotide sequence for use in cell therapy, such as in chimeric antigen receptor T cell (CAR-T) immunotherapy, etc.

The pharmaceutical composition of the present invention can be directly used for binding to CLL1 protein molecules, and thus can be used for the prevention and treatment of CLL1-related diseases. In addition, other therapeutic agents may also be used at the same time.

The pharmaceutical composition of the present invention contains a safe and effective amount (such as 0.001wt% to 99wt%, preferably 0.01wt% to 90wt%, and more preferably 0.1wt% to 80wt%) of the monoclonal antibody (or a conjugate thereof) of the present invention as described above, and a pharmaceutically acceptable carrier or excipient. Such a carrier includes (but is not limited to): saline, buffer, glucose, water, glycerol, ethanol, and a combination thereof. The pharmaceutical formulation should match the mode of administration. The pharmaceutical composition of the present invention may be formulated in the form of an injection prepared, for example, by a conventional method using physiological saline or an aqueous solution containing glucose and other adjuvants. The pharmaceutical composition, such as an injection or a solution, is desirably manufactured under a sterile condition. The administration dosage of the active ingredient is a therapeutically effective amount, such as about 1 µg/kg body weight to about 5 mg/kg body weight per day. In addition, the polypeptide of the present invention may also be used in conjunction with other therapeutic agents.

When the pharmaceutical composition is used, a safe and effective amount of the pharmaceutical composition is administered to a mammal. The safe and effective amount is typically at least about 10 mg/kg body weight, and in most cases does not exceed about 50 mg/kg body weight. Preferably, the dosage is about 10 mg/kg body weight to about 20 mg/kg body weight. Of course, for specific dosages, factors such as the route of administration and the patient's health status should also be considered, all of which are within the skill of an experienced physician.

### Detection use and kit

The antibody of the present invention can be used for detection applications, such as for detecting a sample to provide diagnostic information.

In the present invention, samples (sampled objects) used include cells, tissue samples, and biopsy specimens. The term "biopsy" used in the present invention should include all types of biopsies known to those skilled in the art. Therefore, the biopsies used in the present invention may include tissue samples prepared, for example, by endoscopic methods or by organ puncture or needle biopsy.

The samples used in the present invention include fixed or preserved cell or tissue samples.

The present invention further provides a kit containing the antibody (or a fragment thereof) of the present invention. In a preferred embodiment of the present invention, the kit further comprises a container, a user manual, a buffer, etc. In a preferred embodiment, the antibody of the present invention may be immobilized on a detection plate.

### Main advantages of the present invention include:

(a) The single domain antibody (VHH domain) against CLL1 of the present invention has high specificity and affinity.
(b) The CLL1 CAR T cells of the present invention exhibit high specific in vitro cytotoxicity against CLL1 positive target cells.
(c) The CLL1 CAR T cells of the present invention can effectively inhibit the growth of CLL1 positive tumors in vivo and exhibit long-lasting anti-tumor effects.
(e) The antibody of the present invention can be used to specifically detect a CLL1 protein.

The present invention is further described below in conjunction with specific examples. It should be understood that these examples are only used to illustrate the present invention and are not used to limit the scope of the present invention. Experimental methods for which specific conditions are not indicated in the following examples are usually carried out in accordance with conventional conditions, for example, as described in Sambrook et al., Molecular Cloning: A Laboratory Manual (New York: Cold Spring Harbor Laboratory Press, 1989), or according to the conditions recommended by the manufacturer. Unless otherwise specified, percentages and parts are percentages by weight and parts by weight.

### Example 1. Screening for alpaca antibodies

Single-domain antibodies (i.e., alpaca antibodies or VHH antibodies) were obtained by screening a phage antibody library. The process of establishing an alpaca antibody library is briefly described as follows: An antigen was mixed and emulsified with an adjuvant and was used to immunize an alpaca by hypodermic injection three times successively. After immunization for three times, a blood sample was taken and PBMCs were separated. Total RNA of the PBMCs was extracted and reverse transcribed into cDNA. VHH was amplified through nested PCR, and the VHH fragments were inserted into phagemids. The phagemids were amplified and harvested to obtain an antibody library.

CLL1 antibody screening was performed using protein panning. The method was as follows: The CLL1 antigen was coated overnight. On the next day, after blocking, the antibody library was added and incubation was performed for 1 hour. After the incubation was completed, washing was performed 8 to 10 times. Then, the bound phage was eluted and amplified, and the amplified phage was separated and purified for the next round of panning.

After enrichment was observed during the screening, host bacteria were infected using the enriched phages and plated onto a resistant plate, and ≥ 96 monoclones/library were randomly picked for culture. Supernatants from overnight cultures were collected, and positive monoclones were identified by a phage ELISA experiment. Multiple monoclones were selected for sequencing according to the OD value of the phage ELISA positive well (Pro) and the difference (Pro/PVA) from the control well (PVA). Further, sequences proved to be abnormal in the sequencing were eliminated and clones having the same sequence were combined.

### Results

Ten single-domain antibodies that bind to CLL1 were finally obtained according to the order of affinity of the positive monoclones and the frequency of occurrence of enriched antibodies. The SEQ ID NOs corresponding to the amino acid sequences and CDRs thereof are as described in Table 1, Table 2 and Table 3 below.

**Table 1 Amino acid sequence of anti-CLL1 single-domain antibodies**

| Antibody No. | Amino acid sequence (CDRs underlined) | SEQ ID No: |
|---|---|---|
| C1 | QVQLVESGGGLVQPGGSLRLSCAASGFTFSSKTMGWARQVP GKAIEWLSGIYSDGSTYYADSVKGRFTISRDNAKNMVYLQMNS LKPEDTAVYYCATEDLTSYGRGTQVTVSS | 1 |
| C2 | QVQLVESGGGLVQPGGSLRLSCAASGFTFSSYAMGWARQVP GKEVEWVSGIYSDGSTYYANSVKGRFTISRDNAKNTMYLQMN SLKPEDTAVYHCATDPTVHSDLPVWGQGTLVTVSS | 2 |
| C3 | QVQLVESGGGLVQPGGSLRLSCAASGFTFSSYAMGWARQVP GKEVEWVSGIYSDGRTYYADSVKGRFTISRDNAKNEVYLQMN SLKPEDTAVYYCAPEPGQGVGDYWGQGTQVTVSS | 3 |
| C4 | QVQLVESGGGLVQPGGSLRLSCAASGFTFSSYAMGWARQVP GKDVEWVSGIYSDGSTNYADSVKGRFTISRDNAKNTVYLQMN SLRPEDTAVYHCATDPTVHSDLPVWGQGTLVTVSS | 4 |
| C5 | QVQLVESGGGLVQPGGSLRLSCAASGFTFSSYDMGWARQVP GKAVEWVSGIYSDGSTYYADSVKGRFTISRDNAKNTVYLQMN SLKPEDTAVYYCATDPLTSELRSWGQGTQVTVSS | 5 |
| C6 | QVQLVESGGGLVQPGGSLRLSCTASGFTFSSYAMGWARQVP GKEVEWVSGIYSDGSTNYADSVKGRFTISRDNAKNTVYLQMN SLRPEDTAVYHCATDPTVHSDLPVWGQGTLVTVSS | 6 |
| C7 | QVQLVESGGGLVQPGGSLRLSCAASGFTFSSYAMGWARQVP GKEVEWVSGIYSDGSTNYADSVKGRFTISRDNAKNTVYLQMN SLRPEDTAVYHCATDPTVHSDLPVWGQGTLVTVSS | 7 |
| C8 | QVQLVESGGGLVQPGGSLRLSCAASGFTFSSYAMGWARQVP GKEVEWVSGIYSDGSTNYADSVKGRFTISRDNAKNTVYLQMN SLRPEDTAVYHCATDPTVHSDLPVWGQGTQVTVSS | 8 |
| C9 | QVQLVESGGGLVQPGGSLRLSCAASGFTFSSYAMGWARQVP GKDVEWVSGIYSDGSTYYADSVKGRFTISRYNAKNTVYLQMN SLKPEDTAVYYCATDPTLENQTTAWGQGTQVTVSS | 9 |
| C10 | QVQLVESGGGLVQPGGSLRLACAASGFTFSSYAMGWARQVP GKDVEWVSGIYSDGSTYYADSVKGRFTISRDNAKNTVYLQMN SLKPEDTAVYYCATDPTLENQTTAWGQGTQVTVSS | 10 |

**Table 2 Amino acid sequences of CDRs of anti-CLL1 single-domain antibodies**

| Antibody No. | Full-length sequence | CDR1 | CDR2 | CDR3 |
|---|---|---|---|---|
| C1 | 1 | SKTMG | GIYSDGSTYYADSVKG | EDLTS |
| C2 | 2 | SYAMG | GIYSDGSTYYANSVKG | DPTVHSDLPV |
| C3 | 3 | SYAMG | GIYSDGRTYYADSVKG | EPGQGVGDY |
| C4 | 4 | SYAMG | GIYSDGSTNYADSVKG | DPTVHSDLPV |
| C5 | 5 | SYDMG | GIYSDGSTYYADSVKG | DPLTSELRS |
| C6 | 6 | SYAMG | GIYSDGSTNYADSVKG | DPTVHSDLPV |
| C7 | 7 | SYAMG | GIYSDGSTNYADSVKG | DPTVHSDLPV |
| C8 | 8 | SYAMG | GIYSDGSTNYADSVKG | DPTVHSDLPV |
| C9 | 9 | SYAMG | GIYSDGSTYYADSVKG | DPTLENQTTA |
| C10 | 10 | SYAMG | GIYSDGSTYYADSVKG | DPTLENQTTA |

**Table 3: SEQ ID NOs of anti-CLL1 single-domain antibodies**

| Antibody No. | Full-length sequence | CDR1 | CDR2 | CDR3 |
|---|---|---|---|---|
| C1 | SEQ ID NO: 1 | SEQ ID NO: 11 | SEQ ID NO: 12 | SEQ ID NO: 13 |
| C2 | SEQ ID NO: 2 | SEQ ID NO: 14 | SEQ ID NO: 15 | SEQ ID NO: 16 |
| C3 | SEQ ID NO: 3 | SEQ ID NO: 14 | SEQ ID NO: 17 | SEQ ID NO: 18 |
| C4 | SEQ ID NO: 4 | SEQ ID NO: 14 | SEQ ID NO: 19 | SEQ ID NO: 16 |
| C5 | SEQ ID NO: 5 | SEQ ID NO: 20 | SEQ ID NO: 12 | SEQ ID NO: 21 |
| C6 | SEQ ID NO: 6 | SEQ ID NO: 14 | SEQ ID NO: 19 | SEQ ID NO: 16 |
| C7 | SEQ ID NO: 7 | SEQ ID NO: 14 | SEQ ID NO: 19 | SEQ ID NO: 16 |
| C8 | SEQ ID NO: 8 | SEQ ID NO: 14 | SEQ ID NO: 19 | SEQ ID NO: 16 |
| C9 | SEQ ID NO: 9 | SEQ ID NO: 14 | SEQ ID NO: 12 | SEQ ID NO: 22 |
| C10 | SEQ ID NO: 10 | SEQ ID NO: 14 | SEQ ID NO: 12 | SEQ ID NO: 22 |

Note: These antibodies may have identical CDRs. If the same numbering is used for the same sequence, then the deduplicated numbering is as follows: among them, antibodies C6, C7 and C8 have three identical CDRs; and antibodies C9 and C10 have three identical CDRs.

### Example 2. Cell culture and construction

CLL1-expressing cell lines HL60-LucG cells and KG1-LucG cells and non-CLL1-expressing K562-LucG cells were all cultured using RPMI 1640 culture medium; 293T and wild-type and CLL 1-expressing Hela cells (Hela-WT and Hela-CLL1) were cultured using DMEM culture medium. The above culture media were all supplemented with 10% (v/v) fetal bovine serum, 100 U/ml penicillin and streptomycin, 2 mM L-glutamine, and 1 mM sodium pyruvate. The cells were cultured at 37°C and 5% CO2 under saturated humidity.

The CLL1-expressing Hela cells were a stably transfected cell line obtained by transferring CLL1 antigen through a lentiviral vector, and could specifically express CLL1 protein molecules. The HL60-LucG cells, KG1-LucG cells, and K562-LucG cells were stably transfected cell lines obtained by infecting with a lentivirus expressing firefly luciferase-GFP (T2A ligated) followed by screening.

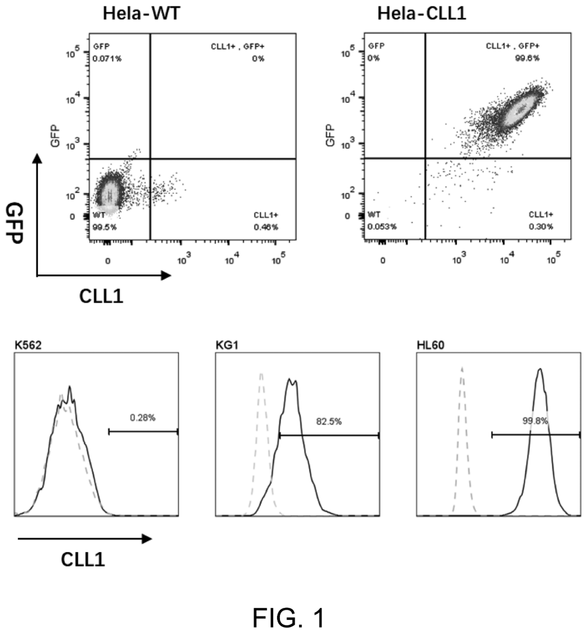

The expression of the antigens on the surface of the target cells used is as shown in Fig. 1. Hela-CLL1 represents CLL1-overexpressing cells, Hela-WT and K562 represent CLL1-negative cells, and KG1 and HL60 represent CLL1-positive cells, the abundance of the CLL1 protein on the surface of HL60 cells being higher than the abundance of the CLL1 protein on the surface of KG1 cells.

### Example 3. Preparation of CLL1 C-CAR T cells

The CLL1 CAR gene consists of a CD8 signal peptide, a VHH or scFv that recognizes CLL1, a CD8 hinge and transmembrane region, a 4-1BB signal region, and a CD3z signal region.

The CLL1 CAR gene was placed under the promoter of EF1α (EF-1α) to form a CLL1 CAR expression vector. The CLL1 CAR expression vectors were respectively numbered C1, C2, C3..., C9, and C10 according to the difference in the VHH sequence. The CLL1 CAR expression vectors constructed using other CLL1-recognizing scFvs were named using their scFv clone numbers, such as M26.

The amino acid sequences of the CLL1 C-CARs of the present invention are as shown in SEQ ID NOs: 32-41, and the amino acid sequence of the scFv-based M26 CAR is as shown in SEQ ID NO: 42.

2.5×10⁶ 293T cells were seeded in DMEM culture medium containing 10% FBS in a 150 cm₂ dish, and the cells were cultured overnight at 37°C and 5% CO₂ under saturated humidity prior to transfection.

On the following day, a helper plasmid and the CLL1 CAR expression vector were added to a centrifuge tube containing 13.8 mL of OptiMEM culture medium, and then 80 µg of PEI was added to the tube to obtain a mixture. The mixture was left to stand at room temperature for 20 minutes, and then supplemented with 12 mL of OptiMEM culture medium to obtain a transfection culture medium. For transfection, after removing the culture medium, the 293T cells were incubated with the transfection culture medium for 4 to 6 hours, and then the transfection culture medium was replaced with 20 mL of DMEM culture medium containing 2% FBS. After 72 hours, the culture medium was collected and centrifuged at 3000 g and 4°C for 15 min. The supernatant was further centrifuged at 27000 g and 4°C for 2 hours. The pellet was collected and resuspended with 400 µL of pre-cooled X-VIVO culture medium to obtain a CLL1 CAR lentiviral suspension and kept at 4°C overnight. On the following day, the virus suspension was aliquoted for further use.

T cells were all cultured in X-VIVO culture medium supplemented with 1% human albumin, 4% serum replacement and 300 IU/mL human interleukin-2 (hIL-2). Pan T cells and CD3/CD28 Dynabeads at a ratio of 1:1 (CD3/CD28 Dynabeads: T cells) and 300 IU/mL of IL2 were incubated together to activate the cells. After 2 days, the Dynabeads were removed using a magnetic column, and then the T cells were transfected with the CLL1 CAR virus at a cell density of 1×10⁶ cells/mL at 37°C. The transfected cells were further cultured for expansion. Three days after infection and before cryopreservation, samples were taken to determine the number of the cells, and the proportion of the CLL1 CAR-positive cells was determined using CLL1 antigen, that is, the CAR positive rate of the T cells were determined. Half of the culture medium was replaced every 2 to 3 days, and the CLL1 C-CAR T cells were harvested on day 8 after infection.

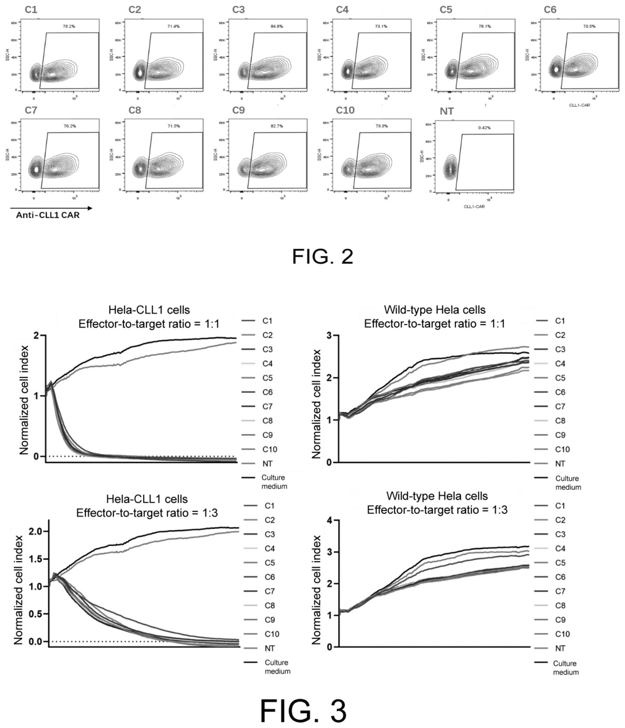

Fig. 2 shows the CAR positive rate of the CLL1-C-CAR-T cells determined using CLL1 antigen. The CAR positive rate is 50% or more in all groups.

### Example 4. In vitro killing by the CLL1 C-CAR T cells

In this example, an in vitro killing experiment was conducted on the CLL1 C-CAR T cells obtained above.

### 4.1 RTCA assay testing

RTCA assay was used to test the cytotoxicity of CAR-T cells against target cells, that is, a Hela cell line overexpressing CLL1.

The results are as shown in Fig. 3. At two effector-target ratios, the NT control groups did not kill the Hela-CLL1 cells, while the CLL1 C-CAR T cells could exert a specific killing effect on CLL1. At the two effector-to-target ratios, the killing effects of the respective groups of CLL1 C-CAR T cells on the CLL1-overexpressing Hela cell line were close to each other.

### 4.2 Luciferase assay testing

Luciferase-labeled tumor target cells were used to test the killing capability. A luciferase gene was transferred into target cells, and after cloning and screening, stably transformed cell lines HL60-LucG and KG1-LucG were obtained. During the experiment, a luciferin substrate was added, and the luciferase reacted with the luciferin to produce fluorescence. The activity of the luciferase could be determined by measuring the intensity of fluorescence, and the killing effect of the respective kinds of CAR-T cells could be obtained by measuring the cell survival rate.

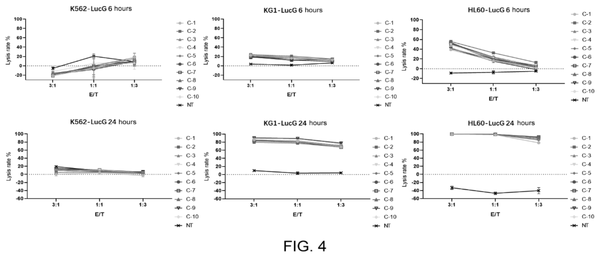

The results are shown in Fig. 4. At the same E:T ratio, the NT cells showed no killing function, the CLL1C-CAR T cells had a dose-dependent killing effect on the HL60-LucG cells and KG1-LucG cells, and the CLL1C-CAR T cells had no killing ability against the K562-LucG cells.

In summary, after the CAR-T cells were co-cultured with target cells (CLL1-overexpressing Hela cells, and CLL1-positive tumor cells, HL60 and KG1 cells), the target cells could be lysed by the CAR-T cells targeting CLL1; and no non-specific cytotoxicity was observed against non-target cells (wild-type Hela cells and CLL1-negative K562 cells).

### Example 5. Recognition of antigen on the surface of target cells by CLL1 antibodies

CLL1 antibodies used were synthesized and purified (the Fc fragment of murine IgG2a was added to the C-terminal of VHH or scFv). These antibodies were serially diluted and used to titrate CLL1 antigen on the surface of HL60 cells, and the ability of these antibodies to recognize antigen on the surface of target cells was analyzed.

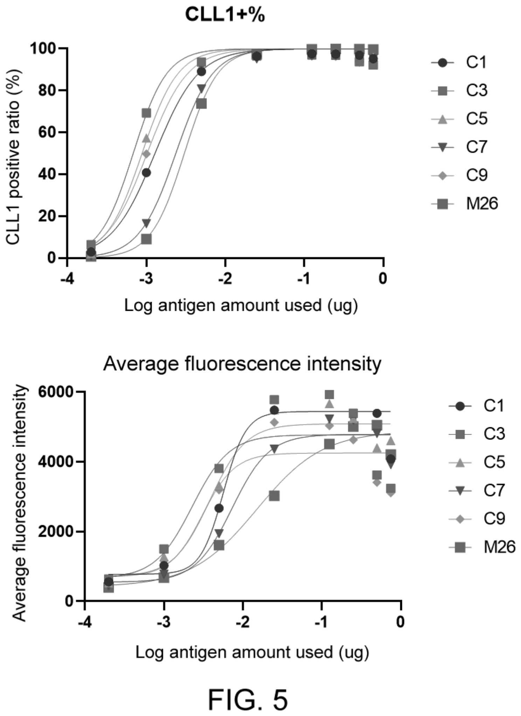

The analysis results are as shown in Fig. 5. Compared with the scFv antibody M26, the single-domain antibodies C1, C3, C5, C7 and C9 of the present invention could recognize CLL1 on the surface of HL60 cells with a higher positive rate, and they had higher average fluorescence intensity than M26 at the same amount of antibody used.

### Example 6. Preparation of CLL1 F-CAR T cells

Peripheral blood was collected from a healthy donor, and PBMCs were isolated by centrifugation at 500-600 g for 20-30 minutes using a density gradient centrifuge. Magnetic beads that bind CD28 antibodies and CD3 antibodies (CD3/CD28 Dynabeads) were used to sort and enrich for T cells. The T cells bound to CD3/CD28 Dynabeads were further incubated with 300 IU/mL IL2 to activate the cells. At the same time, the cells were transfected with CLL1 CAR virus at a cell density of 0.1 to 10×10⁶ cells/mL overnight at 37°C, and on the next day, the cells were washed with physiological saline buffer and then directly frozen for storage. FAST CAR T cells could be obtained without further expansion. In this process, the cells were activated, and these cells were also referred to as CLL1 F-CAR T cells.

The CAR positive rate of the CLL1 F-CAR T cells needed to be titrated after resuscitation. The CLL1 F-CAR T cells were resuscitated and then cultured at 37°C and at a cell density of 1 × 10⁶ cells/mL in the presence of 300 IU/mL IL2. On day 3 after resuscitation, a sample of 1 × 10⁵ cells was taken to determine the proportion of CLL1 CAR-positive cells using CLL1 antigen.

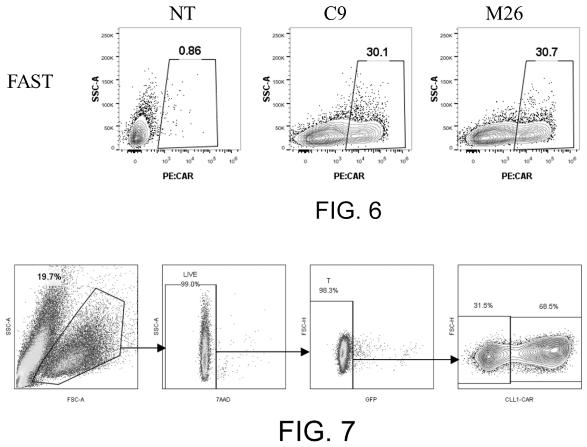

The results are as shown in Fig. 6. The expression of CLL1 CAR could be detected on the surface of the produced CLL1F-CAR Ts, and the positive rate was around 30%.

### Example 7. In vitro multi-round killing by CLL1 C-CAR T and CLL1 F-CAR T cells

On day 1, CLL1F-CAR Ts were resuscitated, and were cultured and incubated together with 300 IU/mL IL2 at 37°C at a cell density of 1 × 10⁶ cells/mL. On day 2, CLL1 C-CAR Ts were resuscitated, and were cultured and incubated together with 300 IU/mL IL2 at 37°C at a cell density of 1 × 10⁶ cells/mL. On day 3, samples of 1 × 10⁵ cells were taken, and the proportion of CLL1 CAR-positive cells was determined using CLL1 antigen.

According to the CAR positive rate of cells as shown in Fig. 6, 2 × 10⁵ CAR-positive CLL1 C-CAR Ts and CLL1 F-CAR Ts were taken and were co-cultured with HL60-LucG (6 × 10⁵ cells, E:T = 1:3) and KG1-LucG (4 × 10⁵ cells, E:T = 1:2), respectively, for multi-round killing analysis.

On day 3 of each round, part of the co-cultured cells were removed for analysis by flow cytometry. The analysis process is shown in Fig. 7, and the specific method is described as follows: A 7AAD-negative viable cell population was circled in a main cell population. Then, T cells (GFP-negative) and target cells (GFP-positive) were distinguished by a GFP signal, and in the T cell population, the CAR positive rate was analyzed using a CLL1 antigen marker. According to the results of flow cytometry and cell counting, the same number of CLL1 CAR-T cells were removed and the tumor cells were supplemented to reach the fixed effector-to-target ratio (the E:T for HL60-LucG was 1:3, and the E:T for KG1-LucG was 1:2) to perform the next round of killing experiment.

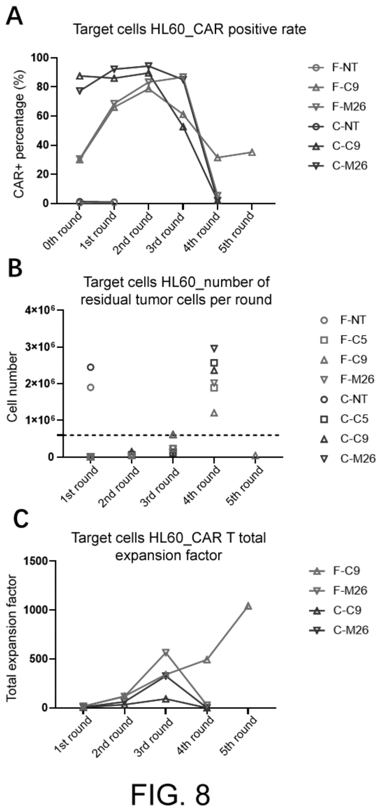

The multi-round killing results against the HL60 target cells are as shown in Fig. 8. Fig. 8A shows the changes in the CAR positive rate of the CLL1 C-CAR T and CLL1 F-CAR T cells during multi-round killing against the HL60; Fig. 8B shows the residual cell number of the target cells (HL60) at the end of each round for the CLL1 C-CAR T and CLL1 F-CAR T cells; and Fig. 8C shows the total expansion times of CAR T cells during multi-round killing against the HL60 by the CLL1 C-CAR T and CLL1 F-CAR T cells. It can be seen from Fig. 8B that the killing ability of the CLL1 F-CAR Ts against the HL60 cells was higher than that of the CLL1 C-CAR Ts; at the end of the fourth round, all the CLL1 C-CAR Ts could no longer inhibit the HL60 cells and the CAR positive rate was significantly reduced (Fig. 8A); and at the end of the fourth round, among the CLL1 F-CAR Ts, C9 had a stronger inhibitory effect on the HL60 than M26, and C9 F-CAR Ts maintained a better CAR positive rate (Fig. 8A). It can be seen from Fig. 8C that during the multiple rounds of killing against the HL60, the expansion of CLL1 F-CAR Ts was better than that of CLL1 C-CAR Ts; and comparison among the CLL1 F-CAR Ts showed that the expansion times of C9 was higher than that of M26.

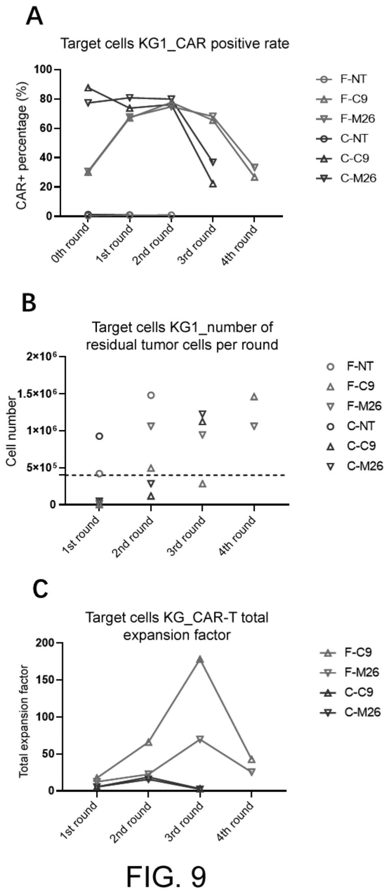

The multi-round killing results against the KG1 target cells are as shown in Fig. 9. Fig. 9A shows the changes in the CAR positive rate of the CLL1 C-CAR T and CLL1 F-CAR T cells during multi-round killing against the KG1; Fig. 9B shows the residual cell number of the target cells (KG1) at the end of each round for the CLL1 C-CAR T and CLL1 F-CAR T cells; and Fig. 9C shows the total expansion times of CAR T cells during multi-round killing against the KG1 by the CLL1 C-CAR T and CLL1 F-CAR T cells. At the end of the third round, none of the CLL1 C-CAR T cells could inhibit the KG1 cells well, but the CLL1 F-CAR T cells could still inhibit the KG1 cells well, and their CAR positive rate was higher than that of the CLL1 C-CAR T cells (Fig. 9A). It can be seen from Fig. 9C that during the multi-round killing against the KG1, the expansion of CLL1 F-CAR T cells was better than that of CLL1 C-CAR T cells; and comparison among the CLL1 F-CAR Ts showed that the expansion times of C9 was higher than that of M26.

### Example 8. NK92 cells and culture method

The complete culture medium for NK92 cells was Alpha MEM supplemented with 12.5% FBS, 12.5% Horse Serum, 1% Pen/strep, 1% sodium pyruvate, 1% L-glutamine, 100U IL2.HL60, Molm13, KG-1, and THP-1, and the complete culture medium for K562 cells was RPMI1640 supplemented with 10% FBS, 1% Pen/strep, 1% sodium pyruvate, and 1% L-glutamine. The cells were cultured at 37°C and 5% CO2 under saturated humidity.

### Example 9. Packaging and transfection of CAR-NK92 lentivirus

293T cells were resuscitated and seeded into a 150 cm² culture dish, and cultured in a CO2 incubator for 72 h. After two passages, the cells were seeded into a 150 cm² culture dish for transfection. A four-plasmid system composed of a lentiviral expression vector, helper plasmid gag/pol, Rev, and VSV-G was mixed with PEI transfection reagent, and then added to a certain volume of serum-free DMEM. The mixture was mixed uniformly and left to stand for 15 min. The resulting mixed solution was added to a 150 cm2 culture dish on which the 293T cells were spread. The cells were gently mixed uniformly, and cultured in a 5% CO₂ cell incubator at 37°C for 6 h. After 6 h, the culture medium was replaced with fresh culture medium and the culture was continued, and after 48 h and 72 h, the lentiviral culture supernatant was collected for infection. The harvested supernatant was transferred to a centrifuge tube and centrifuged at 4000 rpm for 10 min with an acceleration of 9 and a deceleration of 9 to remove cell debris. All the centrifuged LVV supernatants were transferred to a 0.45 µm filter for clarification by filtration, and the filtrates were transferred to new centrifuge tubes. The clarified lentiviral supernatants were added to ultracentrifuge tubes and balanced on a balance. The balanced ultracentrifuge tubes were placed in hanging cups, the hanging cup were placed at corresponding positions of the rotor, and then placed together in an ultracentrifuge for centrifugation. The centrifugation temperature was 4°C, the rotation speed was 100000 g, the centrifugation time was 90 min, and the acceleration and deceleration were set to the highest. After the ultracentrifugation was completed, the supernatants were discarded, and 0.5 mL of culture medium was added to each tube for resuspension at 2°C to 8°C for 2 h. The harvested virus was added to 1e6 NK92 and mixed in a 24-well plate, and cultured in a CO₂ incubator for 72 h.

### Example 10. CAR-NK flow cytometry detection and sorting

The cell suspension was removed, washed with DPBS three times, and centrifuged at 300 g for 5 min. The supernatant was discarded, an antibody mixed solution (anti-FLAG-APC, 1:100 in DPBS) was added. The mixture was mixed uniformly and placed at 4°C for staining for 30 min. The cells were washed with DPBS three times, and centrifuged at 300 g for 5 min. After resuspension in DPBS, the cells were used for flow cytometry detection and sorting. During sorting, PE-positive cells were selected and collected in a 5 ml flow cytometry tube. After the sorting was completed, the cells were centrifuged at 300 g for 5 min, and the supernatant was discarded. The cells were resuspended in a preheated culture medium and placed in a cell culture incubator at 37°C and 5% CO2.

The sorted CAR-NK92 cells were stained with anti-FLAG-APC antibody, and the expression of CAR was detected by flow cytometry.

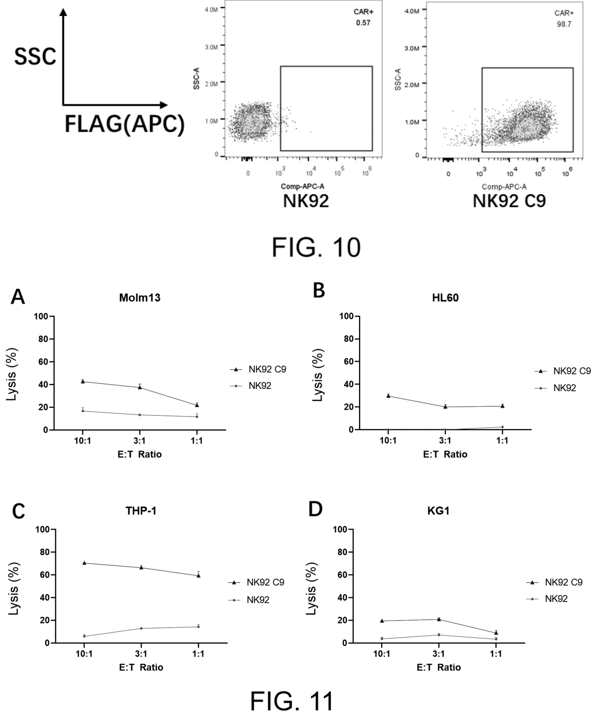

The results of the flow cytometry detection are as shown in Fig. 10. The CAR positive rate of the sorted NK92 C9 cells reached 98% or more.

### Example 11. CAR-NK killing experiment

Luciferase-labeled tumor target cells were used to test the killing capability. A luciferase gene was transferred into target cells to obtain stably transformed cell lines of Molm13, HL60, THP-1 and KG1 expressing the luciferase gene. During the experiment, a luciferin substrate was added, and the luciferase reacted with the luciferin to produce fluorescence. The activity of the luciferase could be determined by measuring the intensity of fluorescence, and the killing effect of the effector cells could be obtained by measuring the cell survival rate.

As shown in Fig. 11, NK92 C9 showed more pronounced cytotoxicity against all target cells compared to NK92, suggesting CAR-mediated killing activity.

### Example 12. Multi-round CAR-NK killing experiment

On the day before the experiment, the fluorescent target cells THP1 were resuspended and counted. The cell density was adjusted to 8 × 10⁴/ml, and the cells were plated on a 96-well plate at 100 ul per well. On the day of the experiment, corresponding numbers of NK92 and CAR-NK92 cells were added to the wells. The plate was placed in a Cellcyte to start the experiment. Imaging was performed every 4 hours in bright field and fluorescence channels. Every two days were taken as one round. After one round of killing was completed, part of the supernatant was removed, and the cells were transferred to a new plate on which the target cells had been spread. The plate was placed in the Cellcyte to start a new round of killing. Killing Index is calculated as the decrease in the number of fluorescent cells after making adjustments for the zero-crossing moment and for normal cell growth variation.

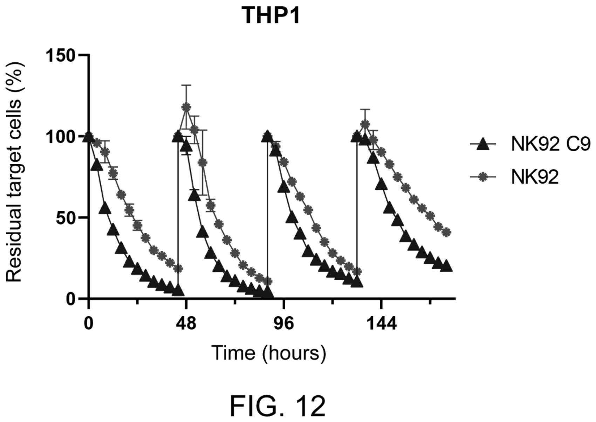

The results of the multi-round killing experiment are as shown in Fig. 12. NK92 C9 cells showed a stronger killing ability than NK92 cells, suggesting that the CAR-mediated killing was continuously effective.

| Sequence information numbering | **Description** | **Sequence** |
|---|---|---|
| **1** | Amino acid sequence of C1 alpaca antibody | QVQLVESGGGLVQPGGSLRLSCAASGFTFSSKTMGWARQVPGKA IEWLSGIYSDGSTYYADSVKGRFTISRDNAKNMVYLQMNSLKPEDT AVYYCATEDLTSYGRGTQVTVSS |
| **2** | Nucleotide sequence of C1 alpaca antibody | CAAGTGCAACTGGTGGAGTCCGGCGGGGGCCTGGTGCAACCC GGCGGCTCCCTGAGACTGAGCTGCGCCGCTAGCGGCTTCACC TTCAGCAGCAAGACCATGGGCTGGGCTAGACAAGTGCCCGGCA AGGCCATCGAGTGGCTGAGCGGCATCTACAGCGACGGCAGCA CCTACTACGCCGACAGCGTGAAGGGCAGATTCACCATCAGCAG AGACAACGCCAAGAACATGGTGTACCTGCAGATGAACAGCCTG AAGCCCGAGGACACCGCCGTGTACTACTGCGCCACCGAGGAC CTGACAAGCTACGGCAGAGGCACCCAAGTGACCGTGAGCAGC |
| **3** | Amino acid sequence of C2 alpaca antibody | QVQLVESGGGLVQPGGSLRLSCAASGFTFSSYAMGWARQVPGKE VEWVSGIYSDGSTYYANSVKGRFTISRDNAKNTMYLQMNSLKPED TAVYHCATDPTVHSDLPVWGQGTLVTVSS |
| **4** | Nucleotide sequence of C2 alpaca antibody | CAAGTGCAACTCGTGGAGAGCGGGGGGGGGCTGGTGCAGCC CGGGGGGAGCCTGAGACTGAGCTGCGCCGCTAGCGGCTTCAC CTTCAGCAGCTACGCCATGGGCTGGGCTAGACAAGTGCCCGGC AAAGAGGTGGAGTGGGTGAGCGGCATCTACAGCGACGGCAGC ACCTACTACGCCAACAGCGTGAAGGGCAGATTCACCATCAGCA GAGACAACGCCAAGAACACCATGTACCTGCAGATGAACAGCCT GAAGCCCGAGGACACCGCCGTGTACCACTGCGCCACAGATCC CACAGTGCACAGCGATCTGCCCGTCTGGGGGCAAGGCACCCT GGTCACCGTGAGCAGC |
| **5** | Amino acid sequence of C3 alpaca antibody | QVQLVESGGGLVQPGGSLRLSCAASGFTFSSYAMGWARQVPGKE VEWVSGIYSDGRTYYADSVKGRFTISRDNAKNEVYLQMNSLKPED TAVYYCAPEPGQGVGDYWGQGTQVTVSS |
| **6** | Nucleotide sequence of C3 alpaca antibody | CAAGTGCAGCTCGTCGAAAGCGGCGGGGGCCTGGTGCAACCT GGCGGGTCCCTGAGACTGAGCTGCGCCGCTAGCGGCTTCACC TTCAGCAGCTACGCCATGGGCTGGGCTAGACAAGTGCCCGGCA AAGAGGTGGAGTGGGTGAGCGGCATCTACAGCGACGGCAGAA CCTACTACGCCGACAGCGTGAAGGGCAGATTCACCATCAGCAG AGACAACGCCAAGAACGAGGTGTACCTGCAGATGAACAGCCTG AAGCCCGAGGACACCGCCGTCTATTACTGTGCCCCCGAGCCTG GCCAAGGCGTGGGGGATTACTGGGGCCAAGGCACCCAAGTGA CCGTGAGCAGC |
| **7** | Amino acid sequence of C4 alpaca antibody | QVQLVESGGGLVQPGGSLRLSCAASGFTFSSYAMGWARQVPGKD VEWVSGIYSDGSTNYADSVKGRFTISRDNAKNTVYLQMNSLRPED TAVYHCATDPTVHSDLPVWGQGTLVTVSS |
| **8** | Nucleotide sequence of C4 alpaca antibody | CAAGTGCAACTCGTGGAGAGCGGGGGGGGGCTGGTGCAGCC CGGGGGGAGCCTGAGACTGAGCTGCGCCGCTAGCGGCTTCAC CTTCAGCAGCTACGCCATGGGCTGGGCTAGACAAGTGCCCGGC AAAGACGTGGAGTGGGTGAGCGGCATCTACAGCGACGGCAGC ACCAACTACGCCGACAGCGTGAAGGGCAGATTCACCATCAGCA GAGACAACGCCAAGAACACCGTGTACCTGCAGATGAACAGCCT GAGACCCGAGGACACCGCCGTGTACCACTGCGCCACAGATCC CACAGTGCACAGCGATCTGCCCGTCTGGGGGCAAGGCACCCT GGTCACCGTGAGCAGC |
| **9** | Amino acid sequence of C5 alpaca antibody | QVQLVESGGGLVQPGGSLRLSCAASGFTFSSYDMGWARQVPGK AVEWVSGIYSDGSTYYADSVKGRFTISRDNAKNTVYLQMNSLKPE DTAVYYCATDPLTSELRSWGQGTQVTVSS |
| **10** | Nucleotide sequence of C5 alpaca antibody | CAAGTGCAGCTGGTCGAAAGCGGCGGCGGGCTCGTGCAGCCC GGCGGCAGCCTGAGACTGAGCTGCGCCGCTAGCGGCTTCACC TTCAGCAGCTACGACATGGGCTGGGCTAGACAAGTGCCCGGCA AGGCCGTGGAGTGGGTGAGCGGCATCTACAGCGACGGCAGCA CCTACTACGCCGACAGCGTGAAGGGCAGATTCACCATCAGCAG AGACAACGCCAAGAACACCGTGTACCTGCAGATGAACAGCCTG AAGCCCGAGGACACCGCCGTGTACTACTGCGCCACCGACCCC CTGACAAGCGAGCTGAGAAGCTGGGGCCAAGGCACCCAAGTG ACCGTGAGCAGC |
| **11** | Amino acid sequence of C6 alpaca antibody | QVQLVESGGGLVQPGGSLRLSCTASGFTFSSYAMGWARQVPGKE VEWVSGIYSDGSTNYADSVKGRFTISRDNAKNTVYLQMNSLRPED TAVYHCATDPTVHSDLPVWGQGTLVTVSS |
| **12** | Nucleotide sequence of C6 alpaca antibody | CAAGTGCAACTCGTGGAGAGCGGGGGGGGGCTGGTGCAGCC CGGGGGGAGCCTGAGACTGAGCTGCACCGCTAGCGGCTTCAC CTTCAGCAGCTACGCCATGGGCTGGGCTAGACAAGTGCCCGGC AAAGAGGTGGAGTGGGTGAGCGGCATCTACAGCGACGGCAGC ACCAACTACGCCGACAGCGTGAAGGGCAGATTCACCATCAGCA GAGACAACGCCAAGAACACCGTGTACCTGCAGATGAACAGCCT GAGACCCGAGGACACCGCCGTGTACCACTGCGCCACAGATCC CACAGTGCACAGCGATCTGCCCGTCTGGGGGCAAGGCACCCT GGTCACCGTGAGCAGC |
| **13** | Amino acid sequence of C7 alpaca antibody | QVQLVESGGGLVQPGGSLRLSCAASGFTFSSYAMGWARQVPGKE VEWVSGIYSDGSTNYADSVKGRFTISRDNAKNTVYLQMNSLRPED TAVYHCATDPTVHSDLPVWGQGTLVTVSS |
| **14** | Nucleotide sequence of C7 alpaca antibody | CAAGTGCAACTCGTGGAGAGCGGGGGGGGGCTGGTGCAGCC CGGGGGGAGCCTGAGACTGAGCTGCGCCGCTAGCGGCTTCAC CTTCAGCAGCTACGCCATGGGCTGGGCTAGACAAGTGCCCGGC AAAGAGGTGGAGTGGGTGAGCGGCATCTACAGCGACGGCAGC ACCAACTACGCCGACAGCGTGAAGGGCAGATTCACCATCAGCA GAGACAACGCCAAGAACACCGTGTACCTGCAGATGAACAGCCT GAGACCCGAGGACACCGCCGTGTACCACTGCGCCACAGATCC CACAGTGCACAGCGATCTGCCCGTCTGGGGGCAAGGCACCCT GGTCACCGTGAGCAGC |
| **15** | Amino acid sequence of C8 alpaca antibody | QVQLVESGGGLVQPGGSLRLSCAASGFTFSSYAMGWARQVPGKE VEWVSGIYSDGSTNYADSVKGRFTISRDNAKNTVYLQMNSLRPED TAVYHCATDPTVHSDLPVWGQGTQVTVSS |
| **16** | Nucleotide sequence of C8 alpaca antibody | CAAGTGCAACTCGTGGAGAGCGGGGGGGGGCTGGTGCAGCC CGGGGGGAGCCTGAGACTGAGCTGCGCCGCTAGCGGCTTCAC CTTCAGCAGCTACGCCATGGGCTGGGCTAGACAAGTGCCCGGC AAAGAGGTGGAGTGGGTGAGCGGCATCTACAGCGACGGCAGC ACCAACTACGCCGACAGCGTGAAGGGCAGATTCACCATCAGCA GAGACAACGCCAAGAACACCGTGTACCTGCAGATGAACAGCCT GAGACCCGAGGACACCGCCGTGTACCACTGCGCCACAGATCC CACAGTGCACAGCGATCTGCCCGTCTGGGGGCAAGGCACCCA AGTCACCGTGAGCAGC |
| **17** | Amino acid sequence of C9 alpaca antibody | QVQLVESGGGLVQPGGSLRLSCAASGFTFSSYAMGWARQVPGKD VEWVSGIYSDGSTYYADSVKGRFTISRYNAKNTVYLQMNSLKPED TAVYYCATDPTLENQTTAWGQGTQVTVSS |
| **18** | Nucleotide sequence of C9 alpaca antibody | CAAGTGCAGCTGGTCGAGAGCGGCGGGGGCCTCGTCCAACCC GGCGGCAGCCTGAGACTGAGCTGCGCCGCTAGCGGCTTCACC TTCAGCAGCTACGCCATGGGCTGGGCTAGACAAGTGCCCGGCA AGGACGTGGAGTGGGTGAGCGGCATCTACAGCGACGGCAGCA CCTACTACGCCGACAGCGTGAAGGGCAGATTCACCATCAGCAG ATACAACGCCAAGAACACCGTGTACCTGCAGATGAACAGCCTGA AGCCCGAGGACACCGCCGTGTACTACTGCGCCACCGATCCTAC CCTCGAGAATCAGACAACCGCCTGGGGCCAAGGGACCCAAGT GACCGTGAGCAGC |
| **19** | Amino acid sequence of C10 alpaca antibody | QVQLVESGGGLVQPGGSLRLACAASGFTFSSYAMGWARQVPGKD VEWVSGIYSDGSTYYADSVKGRFTISRDNAKNTVYLQMNSLKPED TAVYYCATDPTLENQTTAWGQGTQVTVSS |
| **20** | Nucleotide sequence of C10 alpaca antibody | CAAGTGCAACTGGTCGAAAGCGGGGGCGGCCTGGTGCAGCCC GGCGGCAGCCTGAGACTGGCCTGCGCCGCTAGCGGCTTCACC TTCAGCAGCTACGCCATGGGCTGGGCTAGACAAGTGCCCGGCA AGGACGTGGAGTGGGTGAGCGGCATCTACAGCGACGGCAGCA |
| | | CCTACTACGCCGACAGCGTGAAGGGCAGATTCACCATCAGCAG AGACAACGCCAAGAACACCGTGTACCTGCAGATGAACAGCCTG AAGCCCGAGGACACCGCCGTGTACTACTGCGCCACAGATCCCA CACTGGAGAACCAAACCACCGCTTGGGGGCAAGGCACCCAAG TGACCGTGAGCAGC |
| **21** | Amino acid sequence of M26scFv light chain | DIQMTQSPSSLSASLGERVSLTCRATQELSGYLSWLQQKPDGTIK RLIYAASTLDSGVPKRFSGNRSGSDYSLTISSLESEDFADYYCLQYA IYPYTFGGGTKLEIKR |
| **22** | Nucleotide sequence of M26scFv light chain | GACATTCAGATGACACAGAGCCCTAGCAGCCTGAGCGCTAGCC TGGGCGAGAGAGTGAGCCTGACCTGCAGAGCCACCCAAGAGC TGAGCGGCTACCTGAGCTGGCTGCAGCAGAAGCCTGACGGCA CCATCAAGAGACTGATCTACGCCGCTAGCACCCTGGACAGCGG CGTGCCCAAGAGATTCAGCGGCAACAGAAGCGGCAGCGACTAC AGCCTGACCATCAGCAGCCTGGAGAGCGAGGACTTCGCCGAC TACTACTGCCTGCAGTACGCCATCTACCCCTACACCTTCGGCGG GGGCACCAAGCTGGAGATCAAGAGA |
| **23** | Amino acid sequence of M26scFv heavy chain | EVQLQQSGPELVKPGASVKMSCKASGYTFTSYFIHWVKQKPGQG LEWIGFINPYNDGSKYNEKFKGKATLTSDKSSSTAYMELSSLTSED SAVYYCTRDDGYYGYAMDYWGQGTSVTVSS |
| **24** | Nucleotide sequence of M26scFv heavy chain | GAGGTGCAGCTGCAGCAGAGCGGCCCCGAGCTGGTGAAGCCC GGCGCTAGCGTGAAGATGAGCTGCAAGGCTAGCGGCTACACCT TCACAAGCTACTTCATCCACTGGGTGAAACAGAAGCCCGGCCA AGGCCTGGAGTGGATCGGCTTCATCAACCCCTACAACGACGGC AGCAAGTACAACGAGAAGTTCAAGGGCAAGGCCACCCTCACAA GCGACAAGAGCAGCAGCACCGCCTACATGGAGCTGAGCAGCC TGACAAGCGAAGACAGCGCCGTGTACTACTGCACAAGAGACGA CGGCTACTACGGCTACGCCATGGACTACTGGGGCCAAGGCACA AGCGTCACCGTGAGCAGC |
| **25** | Amino acid sequence of (G4S)3 linker peptide | GGGGSGGGGSGGGGS |
| **26** | Nucleotide sequence of (G4S)3 linker peptide | GGGGGCGGGGGCAGCGGCGGGGGGGGGAGCGGCGGGGGC GGCAGC |
| **27** | Amino acid sequence of M26scFv | EVQLQQSGPELVKPGASVKMSCKASGYTFTSYFIHWVKQKPGQG LEWIGFINPYNDGSKYNEKFKGKATLTSDKSSSTAYMELSSLTSED SAVYYCTRDDGYYGYAMDYWGQGTSVTVSSGGGGSGGGGSGG GGSDIQMTQSPSSLSASLGERVSLTCRATQELSGYLSWLQQKPDG TIKRLIYAASTLDSGVPKRFSGNRSGSDYSLTISSLESEDFADYYCL QYAIYPYTFGGGTKLEIKR |
| **28** | Nucleotide sequence of M26 scFv | GAGGTGCAGCTGCAGCAGAGCGGCCCCGAGCTGGTGAAGCCC GGCGCTAGCGTGAAGATGAGCTGCAAGGCTAGCGGCTACACCT TCACAAGCTACTTCATCCACTGGGTGAAACAGAAGCCCGGCCA AGGCCTGGAGTGGATCGGCTTCATCAACCCCTACAACGACGGC AGCAAGTACAACGAGAAGTTCAAGGGCAAGGCCACCCTCACAA GCGACAAGAGCAGCAGCACCGCCTACATGGAGCTGAGCAGCC TGACAAGCGAAGACAGCGCCGTGTACTACTGCACAAGAGACGA CGGCTACTACGGCTACGCCATGGACTACTGGGGCCAAGGCACA AGCGTCACCGTGAGCAGCGGGGGCGGGGGCAGCGGCGGGGG GGGGAGCGGCGGGGGCGGCAGCGACATTCAGATGACACAGAG CCCTAGCAGCCTGAGCGCTAGCCTGGGCGAGAGAGTGAGCCT GACCTGCAGAGCCACCCAAGAGCTGAGCGGCTACCTGAGCTG |
| | | GCTGCAGCAGAAGCCTGACGGCACCATCAAGAGACTGATCTAC GCCGCTAGCACCCTGGACAGCGGCGTGCCCAAGAGATTCAGC GGCAACAGAAGCGGCAGCGACTACAGCCTGACCATCAGCAGC CTGGAGAGCGAGGACTTCGCCGACTACTACTGCCTGCAGTACG CCATCTACCCCTACACCTTCGGCGGGGGCACCAAGCTGGAGAT CAAGAGA |
| **29** | Amino acid sequence of CD8 signal peptide | MALPVTALLLPLALLLHAARP |
| **30** | Nucleotide sequence of CD8 signal peptide | ATGGCCTTACCAGTGACCGCCTTGCTCCTGCCGCTGGCCTTGC TGCTCCACGCCGCCAGGCCG |
| **31** | Amino acid sequence of Flag tag | DYKDDDDK |
| 32 | Amino acid sequence of CD8 hinge transmembra ne region | TTTPAPRPPTPAPTIASQPLSLRPEACRPAAGGAVHTRGLDFACDIY IWAPLAGTCGVLLLSLVITLYC |
| 33 | Nucleotide sequence of CD8 hinge transmembra ne region | ACAACGACACCAGCACCACGGCCACCCACTCCTGCTCCGACAA TTGCGTCTCAGCCCCTTTCCCTTCGACCCGAAGCTTGTCGCCC TGCTGCGGGAGGAGCGGTCCACACGCGCGGGCTTGACTTCGC TTGCGACATCTACATTTGGGCACCCTTGGCCGGGACATGCGGC GTCTTGCTCCTGAGTCTGGTTATAACGCTGTATTGT |
| 34 | Amino acid sequence of 41BB costimulatory domain | KRGRKKLLYIFKQPFMRPVQTTQEEDGCSCRFPEEEEGGCEL |
| 35 | Nucleotide sequence of 41BB costimulatory domain | AAGCGAGGTCGGAAGAAGCTTTTGTATATCTTTAAACAGCCCTTT ATGAGGCCCGTACAAACCACACAAGAGGAGGATGGGTGCTCAT GCAGATTTCCTGAAGAGGAAGAGGGCGGTTGCGAACTT |
| 36 | Amino acid sequence of CD3ζ | RVKFSRSADAPAYKQGQNQLYNELNLGRREEYDVLDKRRGRDPE MGGKPRRKNPQEGLYNELQKDKMAEAYSEIGMKGERRRGKGHD GLYQGLSTATKDTYDALHMQALPPR |
| 37 | Nucleotide sequence of CD3ζ | AGAGTCAAATTCAGCCGCTCCGCAGATGCACCTGCTTATAAACA GGGTCAGAATCAATTGTATAATGAACTTAATCTCGGGAGGCGCG AGGAGTATGATGTGCTGGACAAGCGACGGGGTCGAGACCCAGA GATGGGCGGTAAACCCCGCCGAAAGAACCCCCAGGAGGGACT GTATAATGAGCTGCAAAAGGACAAAATGGCAGAAGCCTATTCCG AAATAGGGATGAAGGGAGAGCGGCGGCGAGGTAAGGGACATG ACGGTCTTTATCAAGGTCTTAGTACTGCAACTAAGGACACCTATG ACGCGCTGCATATGCAGGCTCTCCCACCTAGATAA |
| 38 | Amino acid sequence of C1 CAR | MALPVTALLLPLALLLHAARPQVQLVESGGGLVQPGGSLRLSCAAS GFTFSSKTMGWARQVPGKAIEWLSGIYSDGSTYYADSVKGRFTIS RDNAKNMVYLQMNSLKPEDTAVYYCATEDLTSYGRGTQVTVSSAA ADYKDDDDKTTTPAPRPPTPAPTIASQPLSLRPEACRPAAGGAVHT RGLDFACDIYIWAPLAGTCGVLLLSLVITLYCKRGRKKLLYIFKQPFM RPVQTTQEEDGCSCRFPEEEEGGCELRVKFSRSADAPAYKQGQN QLYNELNLGRREEYDVLDKRRGRDPEMGGKPRRKNPQEGLYNEL |
| | | QKDKMAEAYSEIGMKGERRRGKGHDGLYQGLSTATKDTYDALHM QALPPR |
| 39 | Nucleotide sequence of C1 CAR | ATGGCCTTACCAGTGACCGCCTTGCTCCTGCCGCTGGCCTTGC TGCTCCACGCCGCCAGGCCGCAAGTGCAACTGGTGGAGTCCG GCGGGGGCCTGGTGCAACCCGGCGGCTCCCTGAGACTGAGCT GCGCCGCTAGCGGCTTCACCTTCAGCAGCAAGACCATGGGCTG GGCTAGACAAGTGCCCGGCAAGGCCATCGAGTGGCTGAGCGG CATCTACAGCGACGGCAGCACCTACTACGCCGACAGCGTGAAG GGCAGATTCACCATCAGCAGAGACAACGCCAAGAACATGGTGT ACCTGCAGATGAACAGCCTGAAGCCCGAGGACACCGCCGTGTA CTACTGCGCCACCGAGGACCTGACAAGCTACGGCAGAGGCAC CCAAGTGACCGTGAGCAGCGCCGCTGCCGACTACAAGGACGA CGACGACAAGACAACGACACCAGCACCACGGCCACCCACTCCT GCTCCGACAATTGCGTCTCAGCCCCTTTCCCTTCGACCCGAAG CTTGTCGCCCTGCTGCGGGAGGAGCGGTCCACACGCGCGGGC TTGACTTCGCTTGCGACATCTACATTTGGGCACCCTTGGCCGGG ACATGCGGCGTCTTGCTCCTGAGTCTGGTTATAACGCTGTATTG TAAGCGAGGTCGGAAGAAGCTTTTGTATATCTTTAAACAGCCCTT TATGAGGCCCGTACAAACCACACAAGAGGAGGATGGGTGCTCA TGCAGATTTCCTGAAGAGGAAGAGGGCGGTTGCGAACTTAGAG TCAAATTCAGCCGCTCCGCAGATGCACCTGCTTATAAACAGGGT CAGAATCAATTGTATAATGAACTTAATCTCGGGAGGCGCGAGGA GTATGATGTGCTGGACAAGCGACGGGGTCGAGACCCAGAGATG GGCGGTAAACCCCGCCGAAAGAACCCCCAGGAGGGACTGTATA ATGAGCTGCAAAAGGACAAAATGGCAGAAGCCTATTCCGAAATA GGGATGAAGGGAGAGCGGCGGCGAGGTAAGGGACATGACGGT CTTTATCAAGGTCTTAGTACTGCAACTAAGGACACCTATGACGCG CTGCATATGCAGGCTCTCCCACCTAGA |
| 40 | Amino acid sequence of C2 CAR | MALPVTALLLPLALLLHAARPQVQLVESGGGLVQPGGSLRLSCAAS GFTFSSYAMGWARQVPGKEVEWVSGIYSDGSTYYANSVKGRFTIS RDNAKNTMYLQMNSLKPEDTAVYHCATDPTVHSDLPVWGQGTLV TVSSAAADYKDDDDKTTTPAPRPPTPAPTIASQPLSLRPEACRPAA GGAVHTRGLDFACDIYIWAPLAGTCGVLLLSLVITLYCKRGRKKLLYI FKQPFMRPVQTTQEEDGCSCRFPEEEEGGCELRVKFSRSADAPA YKQGQNQLYNELNLGRREEYDVLDKRRGRDPEMGGKPRRKNPQ EGLYNELQKDKMAEAYSEIGMKGERRRGKGHDGLYQGLSTATKDT YDALHMQALPPR |
| 41 | Nucleotide sequence of C2 CAR | ATGGCCTTACCAGTGACCGCCTTGCTCCTGCCGCTGGCCTTGC TGCTCCACGCCGCCAGGCCGCAAGTGCAACTCGTGGAGAGCG GGGGGGGGCTGGTGCAGCCCGGGGGGAGCCTGAGACTGAGC TGCGCCGCTAGCGGCTTCACCTTCAGCAGCTACGCCATGGGCT GGGCTAGACAAGTGCCCGGCAAAGAGGTGGAGTGGGTGAGCG GCATCTACAGCGACGGCAGCACCTACTACGCCAACAGCGTGAA GGGCAGATTCACCATCAGCAGAGACAACGCCAAGAACACCATG TACCTGCAGATGAACAGCCTGAAGCCCGAGGACACCGCCGTGT ACCACTGCGCCACAGATCCCACAGTGCACAGCGATCTGCCCGT CTGGGGGCAAGGCACCCTGGTCACCGTGAGCAGCGCCGCTGC CGACTACAAGGACGACGACGACAAGACAACGACACCAGCACCA CGGCCACCCACTCCTGCTCCGACAATTGCGTCTCAGCCCCTTT CCCTTCGACCCGAAGCTTGTCGCCCTGCTGCGGGAGGAGCGG TCCACACGCGCGGGCTTGACTTCGCTTGCGACATCTACATTTGG GCACCCTTGGCCGGGACATGCGGCGTCTTGCTCCTGAGTCTGG TTATAACGCTGTATTGTAAGCGAGGTCGGAAGAAGCTTTTGTATA TCTTTAAACAGCCCTTTATGAGGCCCGTACAAACCACACAAGAG GAGGATGGGTGCTCATGCAGATTTCCTGAAGAGGAAGAGGGCG GTTGCGAACTTAGAGTCAAATTCAGCCGCTCCGCAGATGCACCT |
| | | GCTTATAAACAGGGTCAGAATCAATTGTATAATGAACTTAATCTCG GGAGGCGCGAGGAGTATGATGTGCTGGACAAGCGACGGGGTC GAGACCCAGAGATGGGCGGTAAACCCCGCCGAAAGAACCCCC AGGAGGGACTGTATAATGAGCTGCAAAAGGACAAAATGGCAGAA GCCTATTCCGAAATAGGGATGAAGGGAGAGCGGCGGCGAGGTA AGGGACATGACGGTCTTTATCAAGGTCTTAGTACTGCAACTAAG GACACCTATGACGCGCTGCATATGCAGGCTCTCCCACCTAGA |
| 42 | Amino acid sequence of C3 CAR | MALPVTALLLPLALLLHAARPQVQLVESGGGLVQPGGSLRLSCAAS GFTFSSYAMGWARQVPGKEVEWVSGIYSDGRTYYADSVKGRFTIS RDNAKNEVYLQMNSLKPEDTAVYYCAPEPGQGVGDYWGQGTQV TVSSAAADYKDDDDKTTTPAPRPPTPAPTIASQPLSLRPEACRPAA GGAVHTRGLDFACDIYIWAPLAGTCGVLLLSLVITLYCKRGRKKLLYI FKQPFMRPVQTTQEEDGCSCRFPEEEEGGCELRVKFSRSADAPA YKQGQNQLYNELNLGRREEYDVLDKRRGRDPEMGGKPRRKNPQ EGLYNELQKDKMAEAYSEIGMKGERRRGKGHDGLYQGLSTATKDT YDALHMQALPPR |
| 43 | Nucleotide sequence of C3 CAR | ATGGCCTTACCAGTGACCGCCTTGCTCCTGCCGCTGGCCTTGC TGCTCCACGCCGCCAGGCCGCAAGTGCAGCTCGTCGAAAGCG GCGGGGGCCTGGTGCAACCTGGCGGGTCCCTGAGACTGAGCT GCGCCGCTAGCGGCTTCACCTTCAGCAGCTACGCCATGGGCTG GGCTAGACAAGTGCCCGGCAAAGAGGTGGAGTGGGTGAGCGG CATCTACAGCGACGGCAGAACCTACTACGCCGACAGCGTGAAG GGCAGATTCACCATCAGCAGAGACAACGCCAAGAACGAGGTGT ACCTGCAGATGAACAGCCTGAAGCCCGAGGACACCGCCGTCTA TTACTGTGCCCCCGAGCCTGGCCAAGGCGTGGGGGATTACTGG GGCCAAGGCACCCAAGTGACCGTGAGCAGCGCCGCTGCCGAC TACAAGGACGACGACGACAAGACAACGACACCAGCACCACGG CCACCCACTCCTGCTCCGACAATTGCGTCTCAGCCCCTTTCCCT TCGACCCGAAGCTTGTCGCCCTGCTGCGGGAGGAGCGGTCCA CACGCGCGGGCTTGACTTCGCTTGCGACATCTACATTTGGGCA CCCTTGGCCGGGACATGCGGCGTCTTGCTCCTGAGTCTGGTTA TAACGCTGTATTGTAAGCGAGGTCGGAAGAAGCTTTTGTATATCT TTAAACAGCCCTTTATGAGGCCCGTACAAACCACACAAGAGGAG GATGGGTGCTCATGCAGATTTCCTGAAGAGGAAGAGGGCGGTT GCGAACTTAGAGTCAAATTCAGCCGCTCCGCAGATGCACCTGC TTATAAACAGGGTCAGAATCAATTGTATAATGAACTTAATCTCGGG AGGCGCGAGGAGTATGATGTGCTGGACAAGCGACGGGGTCGA GACCCAGAGATGGGCGGTAAACCCCGCCGAAAGAACCCCCAG GAGGGACTGTATAATGAGCTGCAAAAGGACAAAATGGCAGAAG CCTATTCCGAAATAGGGATGAAGGGAGAGCGGCGGCGAGGTAA GGGACATGACGGTCTTTATCAAGGTCTTAGTACTGCAACTAAGG ACACCTATGACGCGCTGCATATGCAGGCTCTCCCACCTAGA |
| 44 | Amino acid sequence of C4 CAR | MALPVTALLLPLALLLHAARPQVQLVESGGGLVQPGGSLRLSCAAS GFTFSSYAMGWARQVPGKDVEWVSGIYSDGSTNYADSVKGRFTI SRDNAKNTVYLQMNSLRPEDTAVYHCATDPTVHSDLPVWGQGTL VTVSSAAADYKDDDDKTTTPAPRPPTPAPTIASQPLSLRPEACRPA AGGAVHTRGLDFACDIYIWAPLAGTCGVLLLSLVITLYCKRGRKKLL YIFKQPFMRPVQTTQEEDGCSCRFPEEEEGGCELRVKFSRSADA PAYKQGQNQLYNELNLGRREEYDVLDKRRGRDPEMGGKPRRKN PQEGLYNELQKDKMAEAYSEIGMKGERRRGKGHDGLYQGLSTAT KDTYDALHMQALPPR |
| 45 | Nucleotide sequence of C4 CAR | ATGGCCTTACCAGTGACCGCCTTGCTCCTGCCGCTGGCCTTGC TGCTCCACGCCGCCAGGCCGCAAGTGCAACTCGTGGAGAGCG GGGGGGGGCTGGTGCAGCCCGGGGGGAGCCTGAGACTGAGC TGCGCCGCTAGCGGCTTCACCTTCAGCAGCTACGCCATGGGCT GGGCTAGACAAGTGCCCGGCAAAGACGTGGAGTGGGTGAGCG |
| | | GCATCTACAGCGACGGCAGCACCAACTACGCCGACAGCGTGAA GGGCAGATTCACCATCAGCAGAGACAACGCCAAGAACACCGTG TACCTGCAGATGAACAGCCTGAGACCCGAGGACACCGCCGTGT ACCACTGCGCCACAGATCCCACAGTGCACAGCGATCTGCCCGT CTGGGGGCAAGGCACCCTGGTCACCGTGAGCAGCGCCGCTGC CGACTACAAGGACGACGACGACAAGACAACGACACCAGCACCA CGGCCACCCACTCCTGCTCCGACAATTGCGTCTCAGCCCCTTT CCCTTCGACCCGAAGCTTGTCGCCCTGCTGCGGGAGGAGCGG TCCACACGCGCGGGCTTGACTTCGCTTGCGACATCTACATTTGG GCACCCTTGGCCGGGACATGCGGCGTCTTGCTCCTGAGTCTGG TTATAACGCTGTATTGTAAGCGAGGTCGGAAGAAGCTTTTGTATA TCTTTAAACAGCCCTTTATGAGGCCCGTACAAACCACACAAGAG GAGGATGGGTGCTCATGCAGATTTCCTGAAGAGGAAGAGGGCG GTTGCGAACTTAGAGTCAAATTCAGCCGCTCCGCAGATGCACCT GCTTATAAACAGGGTCAGAATCAATTGTATAATGAACTTAATCTCG GGAGGCGCGAGGAGTATGATGTGCTGGACAAGCGACGGGGTC GAGACCCAGAGATGGGCGGTAAACCCCGCCGAAAGAACCCCC AGGAGGGACTGTATAATGAGCTGCAAAAGGACAAAATGGCAGAA GCCTATTCCGAAATAGGGATGAAGGGAGAGCGGCGGCGAGGTA AGGGACATGACGGTCTTTATCAAGGTCTTAGTACTGCAACTAAG GACACCTATGACGCGCTGCATATGCAGGCTCTCCCACCTAGA |
| 46 | Amino acid sequence of C5 CAR | MALPVTALLLPLALLLHAARPQVQLVESGGGLVQPGGSLRLSCAAS GFTFSSYDMGWARQVPGKAVEWVSGIYSDGSTYYADSVKGRFTI SRDNAKNTVYLQMNSLKPEDTAVYYCATDPLTSELRSWGQGTQVT VSSAAADYKDDDDKTTTPAPRPPTPAPTIASQPLSLRPEACRPAAG GAVHTRGLDFACDIYIWAPLAGTCGVLLLSLVITLYCKRGRKKLLYIF KQPFMRPVQTTQEEDGCSCRFPEEEEGGCELRVKFSRSADAPAY KQGQNQLYNELNLGRREEYDVLDKRRGRDPEMGGKPRRKNPQE GLYNELQKDKMAEAYSEIGMKGERRRGKGHDGLYQGLSTATKDTY DALHMQALPPR |
| 47 | Nucleotide sequence of C5 CAR | ATGGCCTTACCAGTGACCGCCTTGCTCCTGCCGCTGGCCTTGC TGCTCCACGCCGCCAGGCCGCAAGTGCAGCTGGTCGAAAGCG GCGGCGGGCTCGTGCAGCCCGGCGGCAGCCTGAGACTGAGC TGCGCCGCTAGCGGCTTCACCTTCAGCAGCTACGACATGGGCT GGGCTAGACAAGTGCCCGGCAAGGCCGTGGAGTGGGTGAGCG GCATCTACAGCGACGGCAGCACCTACTACGCCGACAGCGTGAA GGGCAGATTCACCATCAGCAGAGACAACGCCAAGAACACCGTG TACCTGCAGATGAACAGCCTGAAGCCCGAGGACACCGCCGTGT ACTACTGCGCCACCGACCCCCTGACAAGCGAGCTGAGAAGCTG GGGCCAAGGCACCCAAGTGACCGTGAGCAGCGCCGCTGCCGA CTACAAGGACGACGACGACAAGACAACGACACCAGCACCACG GCCACCCACTCCTGCTCCGACAATTGCGTCTCAGCCCCTTTCC CTTCGACCCGAAGCTTGTCGCCCTGCTGCGGGAGGAGCGGTC CACACGCGCGGGCTTGACTTCGCTTGCGACATCTACATTTGGG CACCCTTGGCCGGGACATGCGGCGTCTTGCTCCTGAGTCTGGT TATAACGCTGTATTGTAAGCGAGGTCGGAAGAAGCTTTTGTATAT CTTTAAACAGCCCTTTATGAGGCCCGTACAAACCACACAAGAGG AGGATGGGTGCTCATGCAGATTTCCTGAAGAGGAAGAGGGCGG TTGCGAACTTAGAGTCAAATTCAGCCGCTCCGCAGATGCACCTG CTTATAAACAGGGTCAGAATCAATTGTATAATGAACTTAATCTCGG GAGGCGCGAGGAGTATGATGTGCTGGACAAGCGACGGGGTCG AGACCCAGAGATGGGCGGTAAACCCCGCCGAAAGAACCCCCA GGAGGGACTGTATAATGAGCTGCAAAAGGACAAAATGGCAGAA GCCTATTCCGAAATAGGGATGAAGGGAGAGCGGCGGCGAGGTA AGGGACATGACGGTCTTTATCAAGGTCTTAGTACTGCAACTAAG GACACCTATGACGCGCTGCATATGCAGGCTCTCCCACCTAGA |
| 48 | Amino acid sequence of C6 CAR | MALPVTALLLPLALLLHAARPQVQLVESGGGLVQPGGSLRLSCTAS GFTFSSYAMGWARQVPGKEVEWVSGIYSDGSTNYADSVKGRFTI SRDNAKNTVYLQMNSLRPEDTAVYHCATDPTVHSDLPVWGQGTL VTVSSAAADYKDDDDKTTTPAPRPPTPAPTIASQPLSLRPEACRPA AGGAVHTRGLDFACDIYIWAPLAGTCGVLLLSLVITLYCKRGRKKLL YIFKQPFMRPVQTTQEEDGCSCRFPEEEEGGCELRVKFSRSADA PAYKQGQNQLYNELNLGRREEYDVLDKRRGRDPEMGGKPRRKN PQEGLYNELQKDKMAEAYSEIGMKGERRRGKGHDGLYQGLSTAT KDTYDALHMQALPPR |
| 49 | Nucleotide sequence of C6 CAR | ATGGCCTTACCAGTGACCGCCTTGCTCCTGCCGCTGGCCTTGC TGCTCCACGCCGCCAGGCCGCAAGTGCAACTCGTGGAGAGCGG GGGGGGGCTGGTGCAGCCCGGGGGGAGCCTGAGACTGAGC TGCACCGCTAGCGGCTTCACCTTCAGCAGCTACGCCATGGGCT GGGCTAGACAAGTGCCCGGCAAAGAGGTGGAGTGGGTGAGCG GCATCTACAGCGACGGCAGCACCAACTACGCCGACAGCGTGAA GGGCAGATTCACCATCAGCAGAGACAACGCCAAGAACACCGTG TACCTGCAGATGAACAGCCTGAGACCCGAGGACACCGCCGTGT ACCACTGCGCCACAGATCCCACAGTGCACAGCGATCTGCCCGT CTGGGGGCAAGGCACCCTGGTCACCGTGAGCAGCGCCGCTGC CGACTACAAGGACGACGACGACAAGACAACGACACCAGCACCA CGGCCACCCACTCCTGCTCCGACAATTGCGTCTCAGCCCCTTT CCCTTCGACCCGAAGCTTGTCGCCCTGCTGCGGGAGGAGCGG TCCACACGCGCGGGCTTGACTTCGCTTGCGACATCTACATTTGG GCACCCTTGGCCGGGACATGCGGCGTCTTGCTCCTGAGTCTGG TTATAACGCTGTATTGTAAGCGAGGTCGGAAGAAGCTTTTGTATA TCTTTAAACAGCCCTTTATGAGGCCCGTACAAACCACACAAGAG GAGGATGGGTGCTCATGCAGATTTCCTGAAGAGGAAGAGGGCG GTTGCGAACTTAGAGTCAAATTCAGCCGCTCCGCAGATGCACCT GCTTATAAACAGGGTCAGAATCAATTGTATAATGAACTTAATCTCG GGAGGCGCGAGGAGTATGATGTGCTGGACAAGCGACGGGGTC GAGACCCAGAGATGGGCGGTAAACCCCGCCGAAAGAACCCCC AGGAGGGACTGTATAATGAGCTGCAAAAGGACAAAATGGCAGAA GCCTATTCCGAAATAGGGATGAAGGGAGAGCGGCGGCGAGGTA AGGGACATGACGGTCTTTATCAAGGTCTTAGTACTGCAACTAAG GACACCTATGACGCGCTGCATATGCAGGCTCTCCCACCTAGA |
| 50 | Amino acid sequence of C7 CAR | MALPVTALLLPLALLLHAARPQVQLVESGGGLVQPGGSLRLSCAAS GFTFSSYAMGWARQVPGKEVEWVSGIYSDGSTNYADSVKGRFTI SRDNAKNTVYLQMNSLRPEDTAVYHCATDPTVHSDLPVWGQGTL VTVSSAAADYKDDDDKTTTPAPRPPTPAPTIASQPLSLRPEACRPA AGGAVHTRGLDFACDIYIWAPLAGTCGVLLLSLVITLYCKRGRKKLL YIFKQPFMRPVQTTQEEDGCSCRFPEEEEGGCELRVKFSRSADA PAYKQGQNQLYNELNLGRREEYDVLDKRRGRDPEMGGKPRRKN PQEGLYNELQKDKMAEAYSEIGMKGERRRGKGHDGLYQGLSTAT KDTYDALHMQALPPR |
| 51 | Nucleotide sequence of C7 CAR | ATGGCCTTACCAGTGACCGCCTTGCTCCTGCCGCTGGCCTTGC TGCTCCACGCCGCCAGGCCGCAAGTGCAACTCGTGGAGAGCG GGGGGGGGCTGGTGCAGCCCGGGGGGAGCCTGAGACTGAGC TGCGCCGCTAGCGGCTTCACCTTCAGCAGCTACGCCATGGGCT GGGCTAGACAAGTGCCCGGCAAAGAGGTGGAGTGGGTGAGCG GCATCTACAGCGACGGCAGCACCAACTACGCCGACAGCGTGAA GGGCAGATTCACCATCAGCAGAGACAACGCCAAGAACACCGTG TACCTGCAGATGAACAGCCTGAGACCCGAGGACACCGCCGTGT ACCACTGCGCCACAGATCCCACAGTGCACAGCGATCTGCCCGT CTGGGGGCAAGGCACCCTGGTCACCGTGAGCAGCGCCGCTGC CGACTACAAGGACGACGACGACAAGACAACGACACCAGCACCA CGGCCACCCACTCCTGCTCCGACAATTGCGTCTCAGCCCCTTT |
| | | CCCTTCGACCCGAAGCTTGTCGCCCTGCTGCGGGAGGAGCGG TCCACACGCGCGGGCTTGACTTCGCTTGCGACATCTACATTTGG GCACCCTTGGCCGGGACATGCGGCGTCTTGCTCCTGAGTCTGG TTATAACGCTGTATTGTAAGCGAGGTCGGAAGAAGCTTTTGTATA TCTTTAAACAGCCCTTTATGAGGCCCGTACAAACCACACAAGAG GAGGATGGGTGCTCATGCAGATTTCCTGAAGAGGAAGAGGGCG GTTGCGAACTTAGAGTCAAATTCAGCCGCTCCGCAGATGCACCT GCTTATAAACAGGGTCAGAATCAATTGTATAATGAACTTAATCTCG GGAGGCGCGAGGAGTATGATGTGCTGGACAAGCGACGGGGTC GAGACCCAGAGATGGGCGGTAAACCCCGCCGAAAGAACCCCC AGGAGGGACTGTATAATGAGCTGCAAAAGGACAAAATGGCAGAA GCCTATTCCGAAATAGGGATGAAGGGAGAGCGGCGGCGAGGTA AGGGACATGACGGTCTTTATCAAGGTCTTAGTACTGCAACTAAG GACACCTATGACGCGCTGCATATGCAGGCTCTCCCACCTAGA |
| 52 | Amino acid sequence of C8 CAR | MALPVTALLLPLALLLHAARPQVQLVESGGGLVQPGGSLRLSCAAS GFTFSSYAMGWARQVPGKEVEWVSGIYSDGSTNYADSVKGRFTI SRDNAKNTVYLQMNSLRPEDTAVYHCATDPTVHSDLPVWGQGTQ VTVSSAAADYKDDDDKTTTPAPRPPTPAPTIASQPLSLRPEACRPA AGGAVHTRGLDFACDIYIWAPLAGTCGVLLLSLVITLYCKRGRKKLL YIFKQPFMRPVQTTQEEDGCSCRFPEEEEGGCELRVKFSRSADA PAYKQGQNQLYNELNLGRREEYDVLDKRRGRDPEMGGKPRRKN PQEGLYNELQKDKMAEAYSEIGMKGERRRGKGHDGLYQGLSTAT KDTYDALHMQALPPR |
| 53 | Nucleotide sequence of C8 CAR | ATGGCCTTACCAGTGACCGCCTTGCTCCTGCCGCTGGCCTTGC TGCTCCACGCCGCCAGGCCGCAAGTGCAACTCGTGGAGAGCG GGGGGGGGCTGGTGCAGCCCGGGGGGAGCCTGAGACTGAGC TGCGCCGCTAGCGGCTTCACCTTCAGCAGCTACGCCATGGGCT GGGCTAGACAAGTGCCCGGCAAAGAGGTGGAGTGGGTGAGCG GCATCTACAGCGACGGCAGCACCAACTACGCCGACAGCGTGAA GGGCAGATTCACCATCAGCAGAGACAACGCCAAGAACACCGTG TACCTGCAGATGAACAGCCTGAGACCCGAGGACACCGCCGTGT ACCACTGCGCCACAGATCCCACAGTGCACAGCGATCTGCCCGT CTGGGGGCAAGGCACCCAAGTCACCGTGAGCAGCGCCGCTGC CGACTACAAGGACGACGACGACAAGACAACGACACCAGCACCA CGGCCACCCACTCCTGCTCCGACAATTGCGTCTCAGCCCCTTT CCCTTCGACCCGAAGCTTGTCGCCCTGCTGCGGGAGGAGCGG TCCACACGCGCGGGCTTGACTTCGCTTGCGACATCTACATTTGG GCACCCTTGGCCGGGACATGCGGCGTCTTGCTCCTGAGTCTGG TTATAACGCTGTATTGTAAGCGAGGTCGGAAGAAGCTTTTGTATA TCTTTAAACAGCCCTTTATGAGGCCCGTACAAACCACACAAGAG GAGGATGGGTGCTCATGCAGATTTCCTGAAGAGGAAGAGGGCG GTTGCGAACTTAGAGTCAAATTCAGCCGCTCCGCAGATGCACCT GCTTATAAACAGGGTCAGAATCAATTGTATAATGAACTTAATCTCG GGAGGCGCGAGGAGTATGATGTGCTGGACAAGCGACGGGGTC GAGACCCAGAGATGGGCGGTAAACCCCGCCGAAAGAACCCCC AGGAGGGACTGTATAATGAGCTGCAAAAGGACAAAATGGCAGAA GCCTATTCCGAAATAGGGATGAAGGGAGAGCGGCGGCGAGGTA AGGGACATGACGGTCTTTATCAAGGTCTTAGTACTGCAACTAAG GACACCTATGACGCGCTGCATATGCAGGCTCTCCCACCTAGA |
| 54 | Amino acid sequence of C9 CAR | MALPVTALLLPLALLLHAARPQVQLVESGGGLVQPGGSLRLSCAAS GFTFSSYAMGWARQVPGKDVEWVSGIYSDGSTYYADSVKGRFTI SRYNAKNTVYLQMNSLKPEDTAVYYCATDPTLENQTTAWGQGTQ VTVSSAAADYKDDDDKTTTPAPRPPTPAPTIASQPLSLRPEACRPA AGGAVHTRGLDFACDIYIWAPLAGTCGVLLLSLVITLYCKRGRKKLL YIFKQPFMRPVQTTQEEDGCSCRFPEEEEGGCELRVKFSRSADA PAYKQGQNQLYNELNLGRREEYDVLDKRRGRDPEMGGKPRRKN |
| | | PQEGLYNELQKDKMAEAYSEIGMKGERRRGKGHDGLYQGLSTAT KDTYDALHMQALPPR |
| 55 | Nucleotide sequence of C9 CAR | ATGGCCTTACCAGTGACCGCCTTGCTCCTGCCGCTGGCCTTGC TGCTCCACGCCGCCAGGCCGCAAGTGCAGCTGGTCGAGAGCG GCGGGGGCCTCGTCCAACCCGGCGGCAGCCTGAGACTGAGCT GCGCCGCTAGCGGCTTCACCTTCAGCAGCTACGCCATGGGCTG GGCTAGACAAGTGCCCGGCAAGGACGTGGAGTGGGTGAGCGG CATCTACAGCGACGGCAGCACCTACTACGCCGACAGCGTGAAG GGCAGATTCACCATCAGCAGATACAACGCCAAGAACACCGTGTA CCTGCAGATGAACAGCCTGAAGCCCGAGGACACCGCCGTGTAC TACTGCGCCACCGATCCTACCCTCGAGAATCAGACAACCGCCT GGGGCCAAGGGACCCAAGTGACCGTGAGCAGCGCCGCTGCC GACTACAAGGACGACGACGACAAGACAACGACACCAGCACCAC GGCCACCCACTCCTGCTCCGACAATTGCGTCTCAGCCCCTTTC CCTTCGACCCGAAGCTTGTCGCCCTGCTGCGGGAGGAGCGGT CCACACGCGCGGGCTTGACTTCGCTTGCGACATCTACATTTGG GCACCCTTGGCCGGGACATGCGGCGTCTTGCTCCTGAGTCTGG TTATAACGCTGTATTGTAAGCGAGGTCGGAAGAAGCTTTTGTATA TCTTTAAACAGCCCTTTATGAGGCCCGTACAAACCACACAAGAG GAGGATGGGTGCTCATGCAGATTTCCTGAAGAGGAAGAGGGCG GTTGCGAACTTAGAGTCAAATTCAGCCGCTCCGCAGATGCACCT GCTTATAAACAGGGTCAGAATCAATTGTATAATGAACTTAATCTCG GGAGGCGCGAGGAGTATGATGTGCTGGACAAGCGACGGGGTC GAGACCCAGAGATGGGCGGTAAACCCCGCCGAAAGAACCCCC AGGAGGGACTGTATAATGAGCTGCAAAAGGACAAAATGGCAGAA GCCTATTCCGAAATAGGGATGAAGGGAGAGCGGCGGCGAGGTA AGGGACATGACGGTCTTTATCAAGGTCTTAGTACTGCAACTAAG GACACCTATGACGCGCTGCATATGCAGGCTCTCCCACCTAGA |
| 56 | Amino acid sequence of C10 CAR | MALPVTALLLPLALLLHAARPQVQLVESGGGLVQPGGSLRLACAAS GFTFSSYAMGWARQVPGKDVEWVSGIYSDGSTYYADSVKGRFTI SRDNAKNTVYLQMNSLKPEDTAVYYCATDPTLENQTTAWGQGTQ VTVSSAAADYKDDDDKTTTPAPRPPTPAPTIASQPLSLRPEACRPA AGGAVHTRGLDFACDIYIWAPLAGTCGVLLLSLVITLYCKRGRKKLL YIFKQPFMRPVQTTQEEDGCSCRFPEEEEGGCELRVKFSRSADA PAYKQGQNQLYNELNLGRREEYDVLDKRRGRDPEMGGKPRRKN PQEGLYNELQKDKMAEAYSEIGMKGERRRGKGHDGLYQGLSTAT KDTYDALHMQALPPR |
| 57 | Nucleotide sequence of C10 CAR | ATGGCCTTACCAGTGACCGCCTTGCTCCTGCCGCTGGCCTTGC TGCTCCACGCCGCCAGGCCGCAAGTGCAACTGGTCGAAAGCG GGGGCGGCCTGGTGCAGCCCGGCGGCAGCCTGAGACTGGCC TGCGCCGCTAGCGGCTTCACCTTCAGCAGCTACGCCATGGGCT GGGCTAGACAAGTGCCCGGCAAGGACGTGGAGTGGGTGAGCG GCATCTACAGCGACGGCAGCACCTACTACGCCGACAGCGTGAA GGGCAGATTCACCATCAGCAGAGACAACGCCAAGAACACCGTG TACCTGCAGATGAACAGCCTGAAGCCCGAGGACACCGCCGTGT ACTACTGCGCCACAGATCCCACACTGGAGAACCAAACCACCGC TTGGGGGCAAGGCACCCAAGTGACCGTGAGCAGCGCCGCTGC CGACTACAAGGACGACGACGACAAGACAACGACACCAGCACCA CGGCCACCCACTCCTGCTCCGACAATTGCGTCTCAGCCCCTTT CCCTTCGACCCGAAGCTTGTCGCCCTGCTGCGGGAGGAGCGG TCCACACGCGCGGGCTTGACTTCGCTTGCGACATCTACATTTGG GCACCCTTGGCCGGGACATGCGGCGTCTTGCTCCTGAGTCTGG TTATAACGCTGTATTGTAAGCGAGGTCGGAAGAAGCTTTTGTATA TCTTTAAACAGCCCTTTATGAGGCCCGTACAAACCACACAAGAG GAGGATGGGTGCTCATGCAGATTTCCTGAAGAGGAAGAGGGCG GTTGCGAACTTAGAGTCAAATTCAGCCGCTCCGCAGATGCACCT |
| | | GCTTATAAACAGGGTCAGAATCAATTGTATAATGAACTTAATCTCG GGAGGCGCGAGGAGTATGATGTGCTGGACAAGCGACGGGGTC GAGACCCAGAGATGGGCGGTAAACCCCGCCGAAAGAACCCCC AGGAGGGACTGTATAATGAGCTGCAAAAGGACAAAATGGCAGAA GCCTATTCCGAAATAGGGATGAAGGGAGAGCGGCGGCGAGGTA AGGGACATGACGGTCTTTATCAAGGTCTTAGTACTGCAACTAAG GACACCTATGACGCGCTGCATATGCAGGCTCTCCCACCTAGA |
| 58 | Amino acid sequence of M26 CAR | MALPVTALLLPLALLLHAARPEVQLQQSGPELVKPGASVKMSCKAS GYTFTSYFIHWVKQKPGQGLEWIGFINPYNDGSKYNEKFKGKATLT SDKSSSTAYMELSSLTSEDSAVYYCTRDDGYYGYAMDYWGQGTS VTVSSGGGGSGGGGSGGGGSDIQMTQSPSSLSASLGERVSLTCR ATQELSGYLSWLQQKPDGTIKRLIYAASTLDSGVPKRFSGNRSGS DYSLTISSLESEDFADYYCLQYAIYPYTFGGGTKLEIKRAAADYKDD DDKTTTPAPRPPTPAPTIASQPLSLRPEACRPAAGGAVHTRGLDFA CDIYIWAPLAGTCGVLLLSLVITLYCKRGRKKLLYIFKQPFMRPVQTT QEEDGCSCRFPEEEEGGCELRVKFSRSADAPAYKQGQNQLYNEL NLGRREEYDVLDKRRGRDPEMGGKPRRKNPQEGLYNELQKDKM AEAYSEIGMKGERRRGKGHDGLYQGLSTATKDTYDALHMQALPPR |
| 59 | Nucleotide sequence of M26 CAR | ATGGCCTTACCAGTGACCGCCTTGCTCCTGCCGCTGGCCTTGC TGCTCCACGCCGCCAGGCCGGAGGTGCAGCTGCAGCAGAGCG GCCCCGAGCTGGTGAAGCCCGGCGCTAGCGTGAAGATGAGCT GCAAGGCTAGCGGCTACACCTTCACAAGCTACTTCATCCACTGG GTGAAACAGAAGCCCGGCCAAGGCCTGGAGTGGATCGGCTTC ATCAACCCCTACAACGACGGCAGCAAGTACAACGAGAAGTTCAA GGGCAAGGCCACCCTCACAAGCGACAAGAGCAGCAGCACCGC CTACATGGAGCTGAGCAGCCTGACAAGCGAAGACAGCGCCGTG TACTACTGCACAAGAGACGACGGCTACTACGGCTACGCCATGGA CTACTGGGGCCAAGGCACAAGCGTCACCGTGAGCAGCGGGGG CGGGGGCAGCGGCGGGGGGGGGAGCGGCGGGGGCGGCAGC GACATTCAGATGACACAGAGCCCTAGCAGCCTGAGCGCTAGCC TGGGCGAGAGAGTGAGCCTGACCTGCAGAGCCACCCAAGAGC TGAGCGGCTACCTGAGCTGGCTGCAGCAGAAGCCTGACGGCA CCATCAAGAGACTGATCTACGCCGCTAGCACCCTGGACAGCGG CGTGCCCAAGAGATTCAGCGGCAACAGAAGCGGCAGCGACTAC AGCCTGACCATCAGCAGCCTGGAGAGCGAGGACTTCGCCGAC TACTACTGCCTGCAGTACGCCATCTACCCCTACACCTTCGGCGG GGGCACCAAGCTGGAGATCAAGAGAGCCGCTGCCGACTACAA GGACGACGACGACAAGACAACGACACCAGCACCACGGCCACC CACTCCTGCTCCGACAATTGCGTCTCAGCCCCTTTCCCTTCGAC CCGAAGCTTGTCGCCCTGCTGCGGGAGGAGCGGTCCACACGC GCGGGCTTGACTTCGCTTGCGACATCTACATTTGGGCACCCTTG GCCGGGACATGCGGCGTCTTGCTCCTGAGTCTGGTTATAACGC TGTATTGTAAGCGAGGTCGGAAGAAGCTTTTGTATATCTTTAAAC AGCCCTTTATGAGGCCCGTACAAACCACACAAGAGGAGGATGG GTGCTCATGCAGATTTCCTGAAGAGGAAGAGGGCGGTTGCGAA CTTAGAGTCAAATTCAGCCGCTCCGCAGATGCACCTGCTTATAA ACAGGGTCAGAATCAATTGTATAATGAACTTAATCTCGGGAGGC GCGAGGAGTATGATGTGCTGGACAAGCGACGGGGTCGAGACC CAGAGATGGGCGGTAAACCCCGCCGAAAGAACCCCCAGGAGG GACTGTATAATGAGCTGCAAAAGGACAAAATGGCAGAAGCCTAT TCCGAAATAGGGATGAAGGGAGAGCGGCGGCGAGGTAAGGGA CATGACGGTCTTTATCAAGGTCTTAGTACTGCAACTAAGGACAC CTATGACGCGCTGCATATGCAGGCTCTCCCACCTAGA |

All documents mentioned herein are incorporated by reference into the present application as if each document were individually incorporated by reference. In addition, it should be understood that, after reading the above teachings of the present invention, those skilled in the art may make various changes or modifications to the present invention, and these equivalent forms also fall within the scope defined by the claims attached to the present application.

## Claims

1. A nanobody that specifically binds to CLL1, **characterized in that** complementary determining regions (CDRs) of a VHH chain of the nanobody comprise the following CDR1, CDR2 and CDR3:
(a) CDR1: the sequence thereof is as shown in SEQ ID NO: 14, 11, or 20;
(b) CDR2: the sequence thereof is as shown in SEQ ID NO: 12, 15, 17, or 19; and
(c) CDR3: the sequence thereof is as shown in SEQ ID NO: 13, 16, 18, 21, or 22.

2. The nanobody according to claim 1, wherein the complementary determining regions (CDRs) of the VHH chain of the nanobody are selected from a group consisting of:
(Y1) CDR1 as shown in SEQ ID NO: 14, CDR2 as shown in SEQ ID NO: 19, and CDR3 as shown in SEQ ID NO: 16;
(Y2) CDR1 as shown in SEQ ID NO: 14, CDR2 as shown in SEQ ID NO: 12, and CDR3 as shown in SEQ ID NO: 22;
(Y3) CDR1 as shown in SEQ ID NO: 14, CDR2 as shown in SEQ ID NO: 15, and CDR3 as shown in SEQ ID NO: 16;
(Y4) CDR1 as shown in SEQ ID NO: 14, CDR2 as shown in SEQ ID NO: 17, and CDR3 as shown in SEQ ID NO: 18;
(Y5) CDR1 as shown in SEQ ID NO: 14, CDR2 as shown in SEQ ID NO: 19, and CDR3 as shown in SEQ ID NO: 16;
(Y6) CDR1 as shown in SEQ ID NO: 11, CDR2 as shown in SEQ ID NO: 12, and CDR3 as shown in SEQ ID NO: 13; and
(Y7) CDR1 as shown in SEQ ID NO: 20, CDR2 as shown in SEQ ID NO: 12, and CDR3 as shown in SEQ ID NO: 21.

3. The nanobody according to claim 1, wherein the amino acid sequence of the nanobody that specifically binds to CLL1 is as shown in any one of SEQ ID NOs: 1-10.

4. A multivalent antibody or multi-epitope-targeting antibody that specifically binds to CLL1, **characterized in that** the multivalent antibody or multi-epitope-targeting antibody comprises at least one antibody component that targets a CLL1 epitope, the antibody component being the anti-CLL1 nanobody according to claim 1.

5. A recombinant protein, **characterized in that** the recombinant protein comprises:
(i) the nanobody that specifically binds to CLL1 according to claim 1, or the multivalent antibody or multi-epitope-targeting antibody that specifically binds to CLL1 according to claim 4; and
(ii) an optional tag sequence that facilitates expression and/or purification.

6. A chimeric antigen receptor (CAR) fusion protein, **characterized in that** the chimeric antigen receptor (CAR) fusion protein comprises, from the N-terminus to the C-terminus:
(i) an antigen-binding domain that specifically binds to CLL1, the antigen-binding domain comprising the nanobody according to claim 1;
(ii) a transmembrane domain;
(iii) at least one co-stimulatory domain; and
(iv) an activation domain.

7. The CAR fusion protein according to claim 6, wherein the CAR has the structure as shown in Formula la:
L-VHH-F-H-TM-C-CD3ζ (la)
where,
each "-" is independently a linker peptide or peptide bond;
L is a signal peptide sequence;
VHH is the antigen-binding domain that specifically binds to CLL1; the amino acid sequence of the VHH is as shown in any one of SEQ ID NOs: 1-10;
F is none or a Flag tag;
H is a hinge region;
TM is a transmembrane domain;
C is a co-stimulatory signal domain; and
CD3ζ is a cytoplasmic signal transduction sequence (including wild-type, or mutant/modification thereof) derived from CD3ζ.

8. The CAR fusion protein according to claim 6, wherein the CAR fusion protein has an amino acid sequence as shown in any one of SEQ ID NOs: 32-41.

9. An antibody-drug conjugate, **characterized in that** the antibody-drug conjugate comprises:
(a) the nanobody according to claim 1, the multivalent antibody or multi-epitope-targeting antibody according to claim 4, or the recombinant protein according to claim 5; and
(b) a conjugated moiety coupled to the antibody moiety, the conjugated moiety being selected from a group consisting of: a detectable label, a drug, a toxin, a cytokine, a radionuclide, an enzyme, or a combination thereof.

10. A polynucleotide, **characterized in that** the polynucleotide encodes a protein selected from a group consisting of: the nanobody according to claim 1, the multivalent antibody or multi-epitope-targeting antibody according to claim 4, the recombinant protein according to claim 5, or the CAR fusion protein according to claim 6.

11. An expression vector, **characterized in that** the expression vector comprises the polynucleotide according to claim 10.

12. A host cell, **characterized in that** the host cell comprises the expression vector according to claim 11, or has the polynucleotide according to claim 10 integrated in a genome thereof, or expresses the nanobody according to claim 1, the multivalent antibody or multi-epitope-targeting antibody according to claim 4, the recombinant protein according to claim 5, or the CAR fusion protein according to claim 6.

13. A pharmaceutical composition, **characterized in that** the pharmaceutical composition comprises:
(i) the nanobody according to claim 1, the multivalent antibody or multi-epitope-targeting antibody according to claim 4, the recombinant protein according to claim 5, or an immune cell that expresses the CAR fusion protein according to claim 6; and
(ii) a pharmaceutically acceptable carrier.

14. A use of the nanobody according to claim 1, the multivalent antibody or multi-epitope-targeting antibody according to claim 4, the recombinant protein according to claim 5, or an immune cell that expresses the CAR fusion protein according to claim 6, **characterized in that** the use is for the preparation of a drug for preventing and/or treating a CLL1-related tumor.

15. A method for treating a disease, **characterized by** comprising administering to a subject in need of treatment: the nanobody according to claim 1, the multivalent antibody or multi-epitope-targeting antibody according to claim 4, the recombinant protein according to claim 5, or an immune cell that expresses the CAR fusion protein according to claim 6.
